(19) Europäisches Patentamt
European Patent Office
Office européen des brevets

(11) Veröffentlichungsnummer: **0 009 153**
**B1**

(12) **EUROPÄISCHE PATENTSCHRIFT**

(45) Veröffentlichungstag der Patentschrift: **14.10.81**

(21) Anmeldenummer: **79103226.1**

(22) Anmeldetag: **31.08.79**

(51) Int. Cl.³: **C 07 C 59/56**, C 07 D 309/10, A 61 K 31/19, A 61 K 31/365, C 07 C 51/08 // C07C59/88, C07C121/76

(54) **Halogenierte Delta-Hydroxycarbonsäurederivate, Verfahren zu deren Herstellung und diese Derivate enthaltende neue Medikamente.**

(30) Priorität: **05.09.78 FR 7825457**
**21.06.79 FR 7915888**

(43) Veröffentlichungstag der Anmeldung: **02.04.80 Patentblatt 80/7**

(45) Bekanntmachung des Hinweises auf die Patenterteilung: **14.10.81 Patentblatt 81/41**

(84) Benannte Vertragsstaaten: **AT BE CH DE FR GB IT NL SE**

(56) Entgegenhaltungen:
**FR-A-2 314 186**
**FR-A-2 373 281**

(73) Patentinhaber: **LABORATOIRES HOECHST S.A., Tour Roussel Nobel 3, Avenue du Général de Gaulle, F-92800 Puteaux (FR)**

(72) Erfinder: **Simon, Pierre, Prof. Dr., Chemin des Lacets 12, F-92310 Sevres (FR)**
Erfinder: **Dreux, Jacques, Prof., Rue Raulin 36, F-69007 Lyon (FR)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

**EP 0 009 153 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Halogenierte Delta-Hydroxycarbonsäurederivate, Verfahren zu deren Herstellung und diese Derivate enthaltende neue Medikamente

Es ist bekannt, daß 6-Methyl-3,4-diphenyl-3,4,5,6-tetrahydropyron-2 der untenstehenden Formel I:

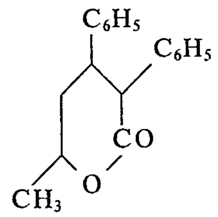

(I)

und die Salze der 5-Hydroxy-2,3-diphenylhexansäure der untenstehenden Formel II:

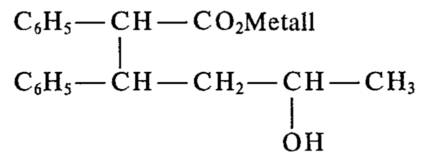

(II)

therapeutische Produkte darstellen, die psychotrope und ganz besonders psychostimulierende Eigenschaften besitzen (vgl. französische Patentschriften 2 277 579, 2 314 186 und 2 373 381).

In Verfolgung der obigen Arbeiten konnten die Erfinder nun zeigen, daß gewisse halogenierte $\delta$-Hydroxycarbonsäurederivate (mit offener oder zyklisierter Kette) psychostimulierende Medikamente mit antidepressiver Wirkung darstellen. Gewisse Stoffe aus dieser wichtigen Gruppe zeigen zwar in pharmakologischer Hinsicht ein sedativartiges Spektrum mit Verminderung der Motilität, aber andere haben es wegen ihrer stimulierenden antidepressiven Wirkung ermöglicht, sehr große Fortschritte in der Behandlung von Depressionen zu machen.

Gegenstand vorliegender Erfindung sind neue halogenierte $\delta$-Hydroxycarbonsäurederivate, welche dadurch gekennzeichnet sind, daß sie der untenstehenden allgemeinen Formel (IIIa):

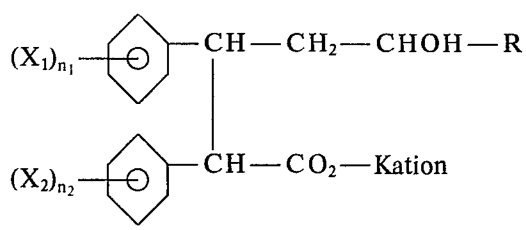

(IIIa)

(offene Form in basischem Medium)

oder der untenstehenden allgemeinen Formel IIIb:

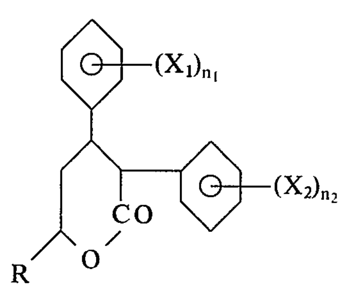

(IIIb)

(zyklisierte Form in saurem Medium)

entsprechen, worin $X_1$ und $X_2$ Halogenatome darstellen, $n_1$ und $n_2$ ganze Zahlen zwischen 0 und 5 bedeuten, jedoch mit dem Vorbehalt, daß im Fall von $n_1 = 0$ $n_2$ von 0 verschieden ist bzw. umgekehrt, R ein Wasserstoffatom oder eine Alkyl- oder Arylgruppe ist und Kation ein pharmazeutisch verträgliches, sich von einer Base herleitendes Ion oder Metallion darstellt.

Gegenstand vorliegender Erfindung ist weiterhin ein Verfahren zur Herstellung von Derivaten der allgemeinen Formeln IIIa und IIIb, welches dadurch gekennzeichnet ist, daß man im Verlauf einer ersten Stufe ein halogeniertes Ketonitril herstellt, welches dann durch Hydrolyse und Reduktion in die

entsprechende Verbindung der allgemeinen Formel III, in basischem Medium in der offenen Form (a) oder in saurem Medium in der zyklisierten Form (b), umgewandelt wird.

Nach einer vorteilhaften Ausführungsform des Verfahrens gemäß dem Gegenstand vorliegender Erfindung wird das halogenierte Ketonitril zunächst zum entsprechenden halogenierten Hydroxynitril reduziert und dann in Gegenwart einer Säure zyklisiert und hydrolysiert, um die Verbindung der allgemeinen Formel IIIb zu erhalten.

Nach einer anderen Ausführungsform des Verfahrens gemäß dem Gegenstand vorliegender Erfindung wandelt man das Ketonitril anfänglich durch Hydrolyse in Gegenwart von Säure in die entsprechende halogenierte Ketosäure um und führt anschließend die Reduktion und Zyklisierung durch, um eine Verbindung der allgemeinen Formel IIIb zu erhalten.

Nach einer weiteren vorteilhaften Ausführungsform des Verfahrens gemäß dem Gegenstand vorliegender Erfindung wird das halogenierte Ketonitril zunächst durch saure Hydrolyse in eine Ketosäure umgewandelt, welche dann mit einer pharmazeutisch verträglichen Base in ein Ketosalz überführt wird, worauf man die Reduktion vornimmt, um eine Verbindung der allgemeinen Formel IIIa zu erhalten.

Nach noch einer weiteren vorteilhaften Ausführungsform des Verfahrens gemäß dem Gegenstand vorliegender Erfindung wandelt man das halogenierte Ketonitril durch Säurebehandlung in Gegenwart eines Alkohols in einen Ketoester um, reduziert diesen dann zum entsprechenden Hydroxyester und hydrolysiert schließlich mit einer pharmazeutisch verträglichen Base, um eine Verbindung der allgemeinen Formel IIIa zu erhalten.

Nach einer vorteilhaften Variante dieser Ausführungsform behandelt man den Ketoester zunächst mit einer pharmazeutisch verträglichen Base zur Bildung eines Ketosalzes und nimmt anschließend die Reduktion vor, um eine Verbindung der allgemeinen Formel IIIa zu erhalten.

Nach noch einer weiteren vorteilhaften Ausführungsform des Verfahrens gemäß dem Gegenstand vorliegender Erfindung überführt man das halogenierte Ketonitril durch alkalische Hydrolyse in das entsprechende Ketosalz und nimmt dann die Reduktion vor.

Nach einer anderen vorteilhaften Ausführungsform des Verfahrens gemäß dem Gegenstand vorliegender Erfindung stellt man das halogenierte Ketonitril durch Einwirkung eines α-äthylenischen Ketons, das der untenstehenden allgemeinen Formel IV

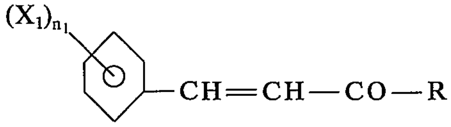

(IV)

entspricht, auf Benzylcyanid der untenstehenden allgemeinen Formel V:

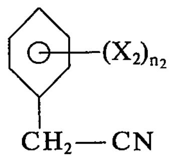

(V)

um eine Verbindung der unten stehenden allgemeinen Formel VI:

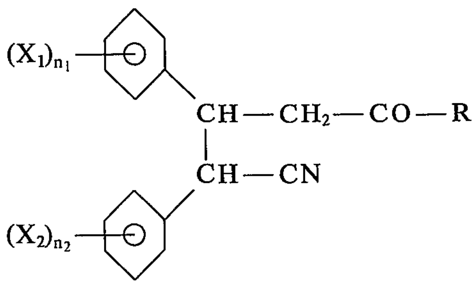

(VI)

worin $X_1$, $X_2$, $n_1$, $n_2$ und R dieselbe Bedeutung wie oben haben, zu erhalten, her.

Nach einer besonders vorteilhaften Ausführungsform des Verfahrens gemäß dem Gegenstand vorliegender Erfindung nimmt man im Verlauf jeder Synthesestufe jeweils mehrere Umkristallisationen aus geeigneten Lösungsmitteln vor, um die trans-(threo)-Derivate von den cis-(erythro)-Derivaten zu trennen oder eines der trans- bzw. cis-Derivate im Gemsich anzureichern.

Nach dieser Verfahrensweise ist es den Erfindern gelungen, die verschiedenen Stereoisomeren dieser Produkte und insbesondere die trans-Form:

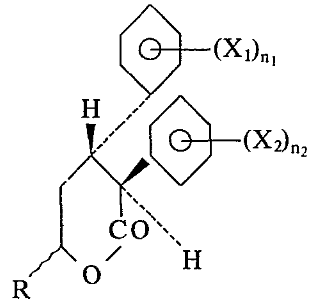

(A)

die cis-Form

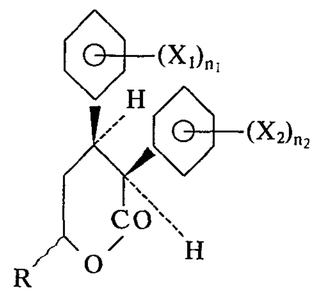

(B)

sowie die beiden Diastereoisomeren bezüglich R der obigen trans- bzw. cis-Formen:

die $\alpha$-Form:

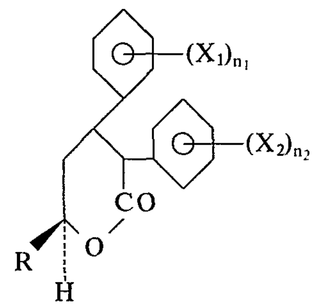

(III$\alpha$)

und die $\beta$-Form:

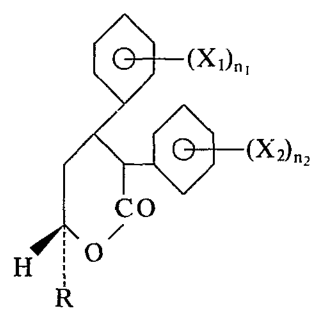

(III$\beta$)

zu trennen.

Nach einer vorteilhaften Ausführungsform des Verfahrens gemäß dem Gegenstand vorliegender Erfindung werden die aus Hydroxynitrilen, Ketonitrilen, Ketosäuren bzw. Ketoestern bestehenden Zwischenprodukte sowie die Pyrone aus polaren Lösungsmitteln und die aus Hydroxyestern bestehenden Zwischenprodukte aus sauerstofffreien polaren Lösungsmitteln umkristallisiert, wogegen die Ketosalze und Hydroxysalze aus mindestens 10% Wasser enthaltenden polaren Lösungsmitteln umkristallisiert werden.

Gemäß einer anderen Ausführungsform des Erfindungsgegenstands erfolgt die Zyklisierung in saurem Medium zur Bildung der Derivate der allgemeinen Formel (III) bei Raumtemperatur, wenn das gesuchte Diastereoisomer die $\beta$-Form ist, bzw. bei Rückflußtemperatur, wenn die Bildung des Diastereoisomeren der $\alpha$-Form erwünscht ist.

Nach einer weiteren Ausführungsform des Erfindungsgegenstands erfolgt die Reduktion der Ketosalze in die entsprechenden Hydroxysalze mit Hilfe von $NaBH_4$ in wäßriger Lösung.

Die Erfinder haben dabei beobachtet, daß das reduzierende System $NaBH_4$/Äthylalkohol eine epimerisierende Rolle spielen kann, denn bei Durchführung der Reduktion der Ketosalze mit $NaBH_4$ in wäßrigem Medium erhält man praktisch reines cis-Isomer und kein Gemisch.

Gegenstand vorliegender Erfindung sind halogenierte $\delta$-Hydroxycarbonsäurederivate der allgemeinen Formeln IIIa und IIIb sowie deren stereochemisch reine Formen der Formeln (A), (B), III$\alpha$ und III$\beta$.

Gegenstand vorliegender Erfindung sind ferner Medikamente, die solche Derivate der Formeln IIIa und IIIb im Gemisch enthalten oder daraus bestehen.

Neben den obenstehenden Varianten umfaßt die Erfindung ferner weitere Varianten, die sich aus der nachfolgenden Beschreibung ergeben werden.

Ganz insbesondere umfaßt vorliegende Erfindung die neuen Medikamente, welche aus den obigen Varianten entsprechenden, halogenierten $\delta$-Hydroxycarbonsäurederivaten bestehen oder diese enthalten, sowie die verschiedenen galenischen Verabreichungsformen dieser Medikamente.

Zum besseren Verständnis sei die Erfindung mit Hilfe der nachfolgenden ergänzenden Beschreibung veranschaulicht, welche sich auf Herstellungsbeispiele für die Verbindungen gemäß vorliegender Erfindung und auch einen Bericht über pharmakologische und pharmakodynamische Versuche mit diesen Verbindungen, die die Wirksamkeit der neuen Medikamente gemäß vorliegender Erfindung als Antidepressiva mit psychostimulierender Wirkung sehr deutlich aufzeigen, bezieht.

Es versteht sich jedoch, daß die weiter unten zu beschreibenden Herstellungsbeispiele sowie der Bericht über pharmakologische und pharmakodynamische Versuche einzig dazu dienen, den Erfindungsgegenstand zu erläutern, jedoch in keiner Weise eine Einschränkung darstellen.

## Beispiel I

Herstellung von trans-4-(4'-Chlorphenyl-6-methyl-3-phenyl-3,4,5,6-tetrahydropyron-2

a) Herstellung von erythro-2-(4'-Chlorphenyl)-4-oxo-1-pentancarbonitril

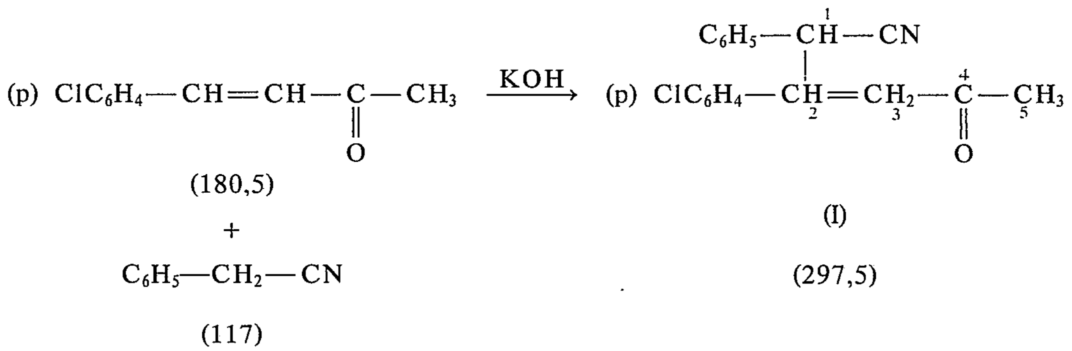


(180,5) + (117) → (I) (297,5)

Man gibt 78 g (0,43 Mol) 1-(4'-Chlorphenyl)-1-butenon-3, 50,5 g (0,43 Mol) Benzylcyanid und 170 $cm^3$ eines Gemischs (30/70) aus Hexan und absolutem Alkohol in einen mit magnetischem Rührer ausgerüsteten 250 $cm^3$-Reaktor. Der Reaktorinhalt wird mit Eis/Salzgemisch auf 0° C gekühlt und dann innerhalb 10 Minuten mit dem Katalysator versetzt (10 $cm^3$ 2n-äthanolische Kalilauge). Während der Katalysatorzugabe stellt sich die Temperatur des Reaktionsmediums auf ungefähr 5° C ein.

Ungefähr 20 Minuten nach beendeter Katalysatorzugabe setzt die Ausfällung des Reaktionsprodukts ein. Eine Stunde nach Beginn der Ausfällung ist das Reaktionsmedium nicht mehr rührbar, und man stellt den Reaktor dann für etwa zwölf Stunden in den Kühlschrank.

Der Reaktorinhalt wird filtriert und zur Entfernung nicht umgesetzter Reagenzien mit 100 $cm^3$ kaltem Hexan und dann zur Entfernung der Kalilauge mit 100 $cm^3$ kaltem Methanol gewaschen. Nach Trocknung im Vakuum gewinnt man 115,5 g weißliche Kristalle des Ketonitrils (I), Schmelzpunkt = 125–128° C (Büchi-Apparat); Ausbeute = 90%. Das Produkt wird einmal aus absolutem Alkohol umkristallisiert.

Schmelzpunkt = 130–132° C; Ausbeute bei der Umkristallisation: 90%.

Analyse: $C_{18}H_{16}ClNO$ (297,5)

| | | | |
|---|---|---|---|
| Berechnet % | C 72,61 | H 5,41 | Cl 11,90 |
| Gefunden % | C 72,66 | H 5,51 | Cl 11;78 |

IR (KBr): $\nu$ C≡N: 2250 $cm^{-1}$; $\nu$ C=1710 $cm^{-1}$.

NMR: $\delta$: 6,85–7,40 ppm, Signal für 9 aromatische Protonen.

(CDCl$_3$) δ: 4,35 ppm, Dublett für ein Proton $H_{(1)}$. J $H_{(1)}$, $H_{(2)}$ = 7 Hz.
δ: 3,65 ppm, Multiplett für ein Proton $H_{(2)}$.
δ: 3,10 ppm, Multiplett für 2 Protonen $H_{(3)}$.
δ: 2,20 ppm, Singulett für 3 Protonen $CH_{3(5)}$ für ein Diastereoisomer.
δ: 2,10 ppm, Singulett für 3 Protonen $CH_{3(5)}$ für das andere Diastereoisomer bezüglich der Kohlenstoffatome 1 und 2, wobei dieses zu weniger als 10% vorliegt. Die Bestimmung der Konfiguration des überwiegenden Diastereoisomeren zeigt übrigens, daß es sich um erythro handelt.

Bei fortgesetztem Umkristallisationen des erhaltenen Produkts aus absolutem Alkohol bis zum konstanten Schmelzpunkt 132°C isoliert man ein einziges reines Diastereoisomer mit erythro-Konfiguration.

b) Herstellung von trans-4-(4'-Chlorphenyl)-6-methyl-3-phenyl-3,4,5,6-tetrahydropyron-2

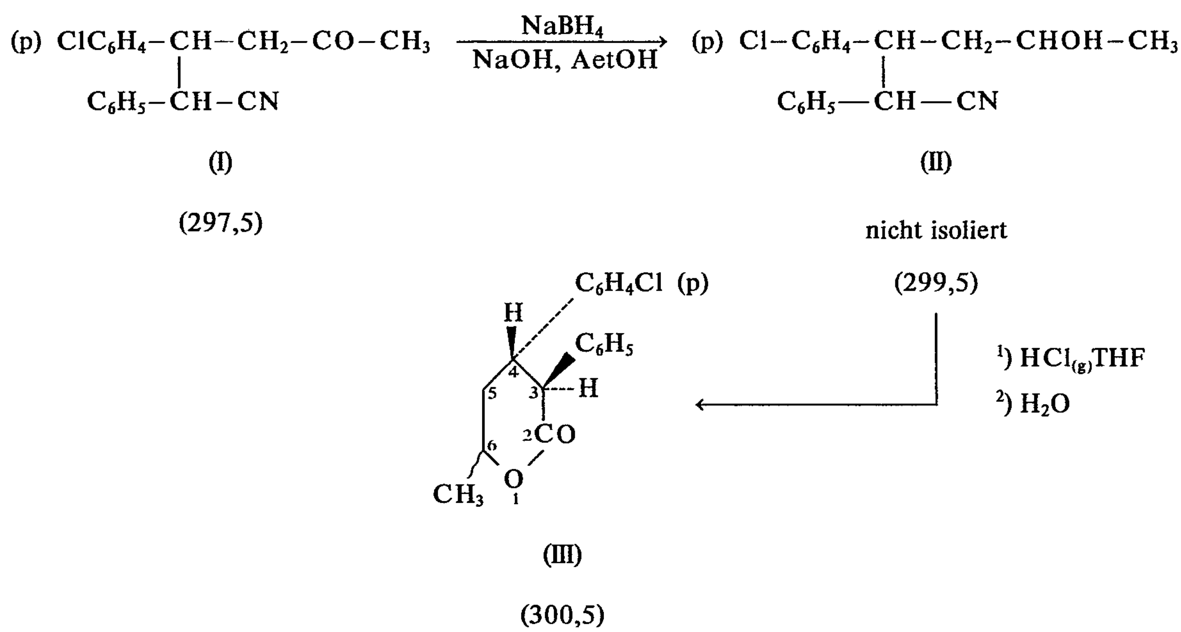


(I) (297,5)

(II) nicht isoliert (299,5)

(III) (300,5)

Eine Suspension von 50 g (0,168 Mol) des oben erhaltenen Ketonitrils (I) in 500 cm$^3$ absolutem Alkohol wird in einem mit magnetischem Rührer ausgerüsteten Einliter-Rundkolben vorgelegt. Dann versetzt man tropfenweise (in ungefähr 15 Minuten) mit einer Lösung von 3,2 g (0,084 Mol) Natriumborhydrid und 0,1 g Ätznatron in 20 cm$^3$ Wasser und rührt 12 Stunden bei Raumtemperatur. Nach Zugabe des Katalysators steigt die Temperatur des Reaktionsmediums auf etwa 30°C, und die Lösung klärt sich. Die Ausfällung der Reaktionsprodukte setzt ungefähr 1 Stunde nach Ende der Katalysatorzugabe ein, und die Temperatur des Reaktionsmediums fällt wieder auf Raumtemperatur.

Der Alkohol wird dann im Vakuum abgedampft und der Rückstand (Nitrilalkohol und Mineralsalze) in 400 cm$^3$ Methylenchlorid aufgenommen. Man extrahiert die Mineralsalze mit 100 cm$^3$ Wasser, wäscht die organische Phase mit Wasser neutral und trocknet sie über Natriumsulfat. Nach Abdampfen des Lösungsmittels erhält man 49,5 g Rohprodukt (Hydroxynitril); Ausbeute = 98%.

Trotz Ausgehens von einem Ketonitril (I) mit erythro-Konfiguration erhält man ein Hydroxynitril, dessen Konfiguration danach als threo bestimmt wird; zugegebenermaßen findet im Verlauf der Herstellung des letzteren eine Epimerisierung statt.

Das erhaltene Produkt wird in 120 cm$^3$ wasserfreiem Tetrahydrofuran (THF) aufgenommen. In diese Lösung läßt man trockenes Chlorwasserstoffgas bis zur Sättigung einperlen, wobei die Temperatur des Mediums bei ungefähr 0°C gehalten wird. Nach 12 Stunden Stehen bei niedriger Temperatur (−10°C) wird die Salzsäure durch einen Luftstrom entfernt und das THF abgedampft. Den Rückstand erhitzt man in 100 cm$^3$ Wasser 1 Stunde zum Rückfluß. Nach Abkühlung wird die organische Phase in 100 cm$^3$ Methylenchlorid aufgenommen und die wäßrige Phase mit demselben Lösungsmittel (2 × 50 cm$^3$) extrahiert. Man vereinigt die organischen Phasen und wäscht sie mit Wasser und dann mit Natriumbicarbonatlösung und schließlich mit Wasser bis zur Neutralität. Die organische Lösung wird über Natriumsulfat getrocknet. Nach Abdampfen des Lösungsmittels kristallisiert man das rohe Pyron aus 100 cm$^3$ absolutem Alkohol um. Dabei isoliert man 30 g Pyron (III). Schmelzpunkt = 139–141°C; Ausbeute = 60%.

Analyse: $C_{18}H_{17}ClO_2$ (300,5)

| | | | |
|---|---|---|---|
| Berechnet % | C 71,88 | H 5,70 | Cl 11,79 |
| Gefunden % | C 71,67 | H 5,59 | Cl 11,81 |

IR (KBr): $\nu$ C=O: 1730 $cm^{-1}$.

NMR:

($CD_3-CO-CD_3$) $\delta$: 7,15 ppm, Signal für 9 aromatische Protonen.
$\delta$: 5,00 ppm, Multiplett für $H_{(6)}$.
$\delta$: 4,20 ppm, Dublett für $H_{(3)}$.
J $H_{(3)}$, $H_{(4)}$ = 11,4 Hz.
$\delta$: 3,90 ppm, Dublett für $H_{(3)}$.
J $H_{(3)}$, $H_{(4)}$ = 11,8 Hz für das andere Isomer.
$\delta$: 3,60 ppm, Multiplett für $H_{(4)}$.
$\delta$: 2,15 ppm, Multiplett für die beiden Protonen $H_{(5)}$ der beiden Isomeren.
$\delta$: 1,45 ppm, Dublett für 3 Protonen $CH_{3(6)}$.
J $CH_3$, $H_{(6)}$ = 6 Hz.
$\delta$: 1,40 ppm, Dublett für 3 Protonen $CH_{3(6)}$ für das andere Isomer.
J $CH_3$, $H_{(6)}$ = 6 Hz.

Die beiden Isomeren liegen zu je 50% vor (nach den Methylsignalen in 6-Stellung gemessen). Die Kupplungskonstante zwischen den Protonen (3) und (4) von 11,4 und 11,8 Hz weist auf eine trans-axiale Konformation der Protonen $H_{(3)}$ und $H_{(4)}$, die beiden Substituenten in 3- und 4-Stellung befinden sich also in trans-äquatorialer Stellung.

Massenspektrum: Zwei Molekülpeaks bei 300 ($Cl^{35}$) und 302 ($Cl^{37}$).

## Beispiel II

### Herstellung von threo-2-(4'-Chlorphenyl)-4-hydroxy-1-phenylpentancarbonitril IIa

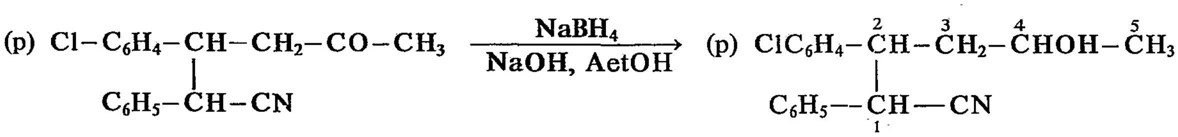


(I) (IIa)

(297,5) (299,5)

Eine Suspension von 40 g (0,134 Mol) des wie in Beispiel I angegeben erhaltenen Ketonitrils (I), Schmelzpunkt = 130—132°C, in 400 $cm^3$ absolutem Alkohol wird in einem mit magnetischem Rührer ausgerüsteten Einliter-Rundkolben vorgelegt. Dann versetzt man tropfenweise (in ungefähr 15 Minuten) mit einer Lösung von 2,55 g (0,067 Mol) Natriumborhydrid und 0,1 g Ätznatron in 20 $cm^3$ Wasser und rührt 12 Stunden bei Raumtemperatur. Nach Zugabe des Katalysators steigt die Temperatur des Reaktionsmediums auf etwa 30°C, und die Lösung klärt sich. Die Ausfällung der Reaktionsprodukte setzt ungefähr 1 Stunde nach Ende der Katalysatorzugabe ein, und die Temperatur des Reaktionsmediums fällt wieder auf Raumtemperatur.

Der Alkohol wird dann im Vakuum abgedampft und der Rückstand (Nitrilalkohol und Mineralsalze) in 400 $cm^3$ Methylenchlorid aufgenommen. Man extrahiert die Mineralsalze mit 100 $cm^3$ Wasser, wäscht die organische Phase mit Wasser neutral und trocknet sie über Natriumsulfat. Nach Abdampfen des Lösungsmittels erhält man 39,5 g Rohprodukt (Hydroxynitril); Ausbeute: 98%.

Das Rohprodukt, ein äquimolares Gemisch der beiden Diastereoisomeren (II$\alpha$) und (II$\beta$) bezüglich des Kohlenstoffatoms 4, wird zweimal aus 100 $cm^3$ Äthylalkohol (95° Gay-Lussac) umkristallisiert. Dabei isoliert man das threo-Isomer (II$\alpha$), Schmelzpunkt = 169—170°C. Ausbeute der Umkristallisation: 75%, berechnet unter Berücksichtigung der Tatsache, daß das erhaltene Produkt aus zwei threo-Diastereoisomeren besteht, die sich in der CHOH-Konfiguration am Kohlenstoffatom (4) unterscheiden, und daß diese Präparation zum Ziel hat, ein einziges dieser Isomeren (II$\alpha$) zu isolieren, um das trans-Pyron oder (III$\alpha$) zu erhalten (siehe Beispiel III weiter unten).

Das Filtrat von der ersten Umkristallisation von (II$\alpha$) ist zurückzubehalten, da es das Diastereoisomer (II$\beta$) enthält, welches später dazu dienen soll, das Pyron (III$\beta$) herzustellen (vgl. Beispiel IV).

Trotz Ausgehens von einem Ketonitril (I) mit erythro-Konfiguration erhält man zwei Pyrone (III$\alpha$) und (III$\beta$) mit trans-Konfiguration, so daß zugegebenermaßen im Verlauf der Herstellung des

Hydroxynitrils eine Epimerisierung stattfindet. Die Konfiguration des erhaltenen Produkts (IIα) wird danach als threo bestimmt.

Analyse: $C_{18}H_{18}ClNO$
(IIα) (299,5)

| | | | |
|---|---|---|---|
| Berechnet % | C 72,24 | H 6,02 | Cl 11,70 |
| Gefunden % | C 72,17 | H 6,07 | Cl 11,64 |

IR (KBr): $\nu$ C≡N: 2250 $cm^{-1}$, $\nu$ O−H: 3525 $cm^{-1}$
(IIα)

NMR: (CDCl₃) (IIα)

- δ: 6,80−7,30 ppm, Signal für 9 aromatische Protonen.
- δ: 4,15 ppm, Dublett für ein Proton $H_{(1)}$. $J_{H(1),H(2)} = 6$ Hz.
- δ: 3,80 ppm, Multiplett für ein Proton $H_{(2)}$.
- δ: 3,20 ppm, Multiplett für ein Proton $H_{(4)}$.
- δ: 1,95 ppm, zwei überlagerte Tripletts für die beiden Protonen $CH_{2(3)}$. $J_{CH_2(3), H(2)} = J_{CH_2(3), H(4)} = 6$ Hz.
- δ: 1,15 ppm, Dublett für 3 Protonen $CH_{3(5)}$. $J_{CH_3, H(4)} = 6$ Hz.

## Beispiel III

Herstellung von trans-4-(4′-Chlorphenyl)-6-methyl-3-phenyl-3,4,5,6-tetrahydropyron-2 IIIα

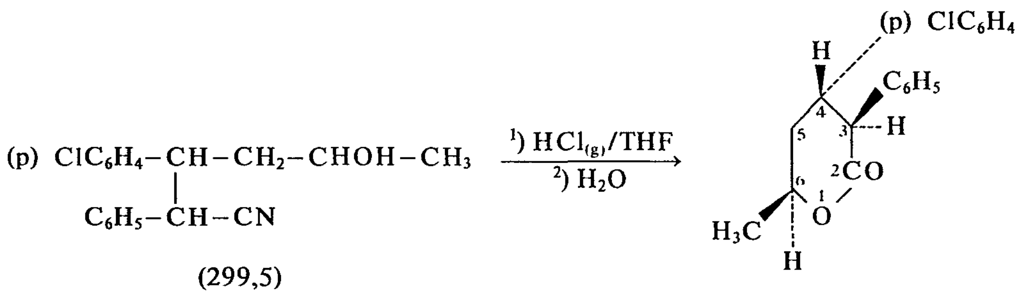


(299,5) (IIa)

(300,5) (IIIa)

Zur Herstellung des Pyrons verfährt man wie in Beispiel I beschrieben. Nach Abdampfen des Lösungsmittels wird das rohe Pyron einmal aus absolutem Alkohol umkristallisiert. Man isoliert ein einziges reines trans-Isomer des Pyrons. Schmelzpunkt = 136−137° C; Ausbeute: 60%

IR (KBr): $\nu$ C=O: 1750 $cm^{-1}$

NMR: ($CD_3COCD_3$)

- δ: 7,10 ppm, Signal für 9 aromatische Protonen
- δ: 4,95 ppm, Multiplett für $H_{(6)}$.
- δ: 4,15 ppm, Dublett für $H_{(3)}$. $J_{H(3), H(4)} = 11,4$ Hz.
- δ: 3,60 ppm, Multiplett für $H_{(4)}$.
- δ: 2,20 ppm, Multiplett für die beiden Protonen $H_{(5)}$.
- δ: 1,42 ppm, Dublett für 3 Protonen $CH_{3(6)}$. J $CH_3$, $H_{(6)}$ = 6 Hz.

## Beispiel IV

### Herstellung von trans-4-(4'-Chlorphenyl)-6-methyl-3-phenyl-3,4,5,6-tetrahydropyron-2 IIIβ

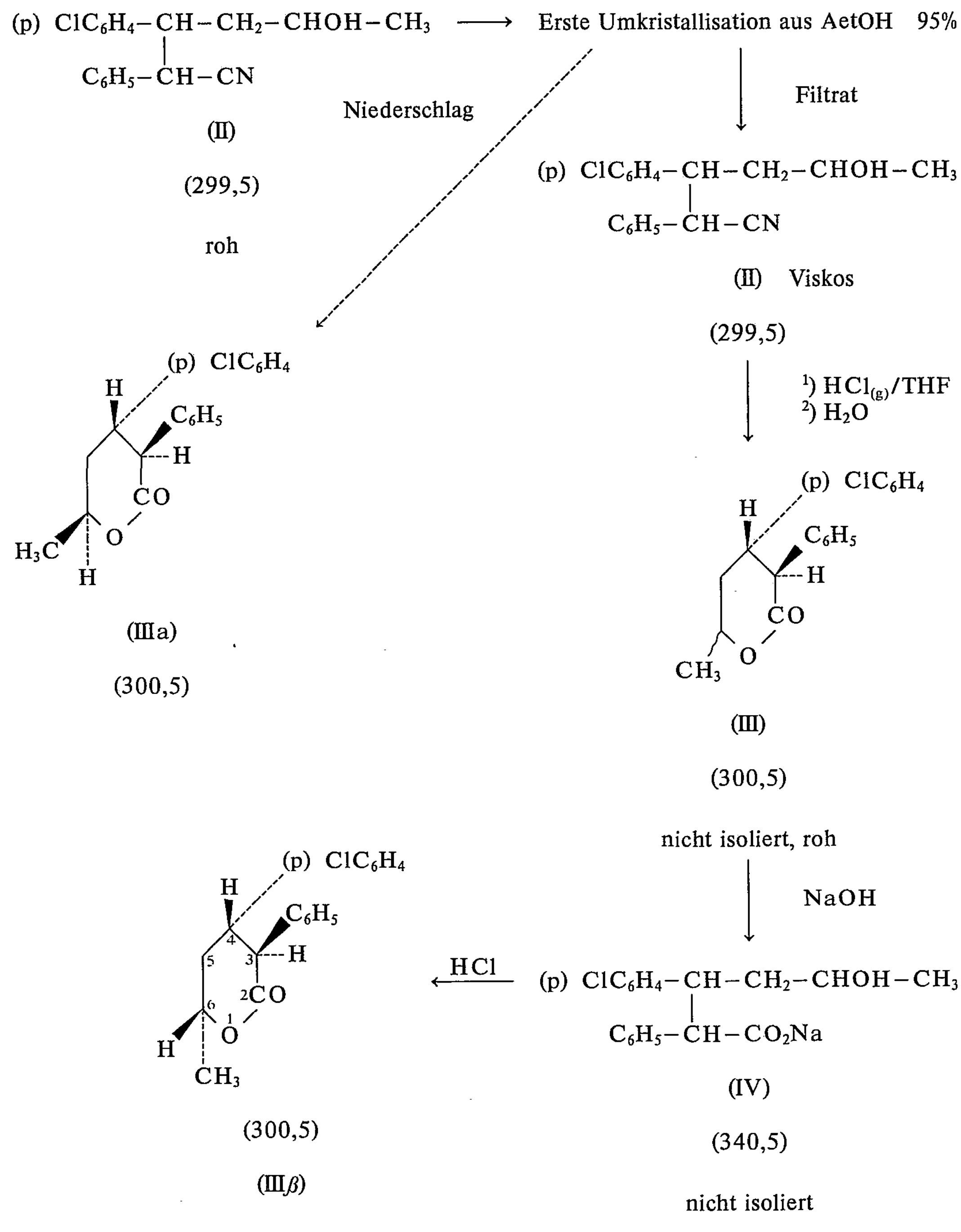


Zur Herstellung des Hydroxynitrils verfährt man wie in Beispiel I beschrieben. Das erhaltene Rohprodukt wird einmal aus Alkohol (95° Gay-Lussac) umkristallisiert. Das Filtrat von dieser Umkristallisation wird zurückgewonnen, und durch Abdampfen des Alkohols erhält man ein viskoses Hydroxynitril (II), das reich an β-Isomer ist (α: 25%, β: 75%). Diesen Prozentsatz erhält man durch Messung der Signale der Methyle in 6-Stellung im NMR-Spektrum des aus diesem Hydroxynitril erhaltenen Pyrons (III).

Die Herstellung des Pyrons wird fortgesetzt, indem man wie in Beispiel I verfährt. Zu 15 g (0,0501 Mol) rohem Pyron (III) gibt man eine Lösung von 2,1 g (0,0528 Mol, 5% Überschuß) Ätznatron in 45 $cm^3$ Wasser und erhitzt eine Stunde unter Rückfluß. Nach Abkühlung wäscht man die wäßrige Lösung zur Entfernung des Unverseifbaren mit Methylenchlorid und säuert die wäßrige Phase mit 10%iger HCl-Lösung an. Man rührt eine Stunde bei Raumtemperatur. Die organische Phase wird in 45 $cm^3$

Methylenchlorid aufgenommen und die wäßrige Phase mit demselben Lösungsmittel (2 × 20 $cm^3$) extrahiert. Die organischen Phasen werden vereinigt und mit Wasser, dann mit Natriumbicarbonatlösung und schließlich mit Wasser bis zur Neutralität gewaschen. Man trocknet die organische Lösung über Natriumsulfat. Nach Abdampfen des Lösungsmittels wird das rohe Pyron (3,5 g, Ausbeute 31%) bis zu konstantem Schmelzpunkt aus absolutem Alkohol umkristallisiert. Man isoliert ein einziges reines trans-Isomer des Pyrons III$\beta$. Schmelzpunkt = 159—160° C. Gesamtausbeute des Verfahrens, bezogen auf das viskose Hydroxynitril (II): 16%.

IR (KBr): $\nu$ C=O: 1725 $cm^{-1}$

| | | |
|---|---|---|
| NMR: | $\delta$: | 7,15 ppm, Signal für 9 aromatische Protonen. |
| ($CD_3COCD_3$) | $\delta$: | 4,90 ppm, Multiplett für $H_{(6)}$. |
| | $\delta$: | 3,95 ppm, Dublett für $H_{(3)}$. |
| | | J $H_{(3)}$, $H_{(4)}$ = 11,8 Hz. |
| | $\delta$: | 3,60 ppm, Multiplett für $H_{(4)}$. |
| | $\delta$: | 2,20 ppm, Multiplett für die beiden Protonen $H_{(5)}$. |
| | $\delta$: | 1,40 ppm, Dublett für 3 Protonen $CH_{3(6)}$. |
| | | J $CH_3$, $H_{(6)}$ = 6 Hz. |

## Beispiel V

Trennung der beiden reinen Diastereoisomeren $\alpha$ und $\beta$ von trans-4-(4'-Chlorphenyl)-6-methyl-3-phenyl-3,4,5,6-pyron-2

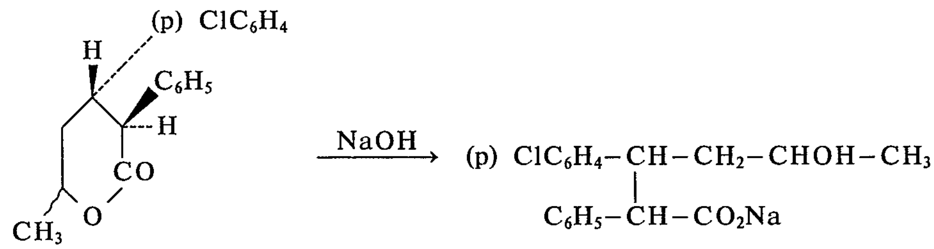


(III) (IV)

(300,5) (340,5)

Umkristallisation aus Wasser

Niederschlag

HCl
Erhitzen am Rückfluß

Filtrat
HCl bei
Raumtemperatur

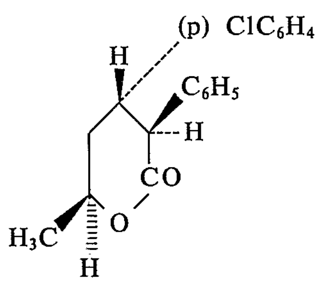

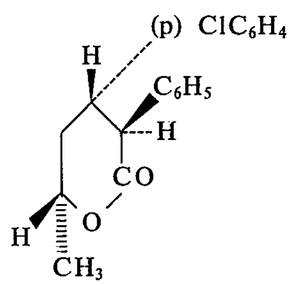

(III$\alpha$) (III$\beta$)

(300,5)

Zu 2 g (0,0066 Mol) des Pyrons (III) gibt man eine Lösung von 0,28 g (0,0070 Mol, 5% Überschuß) Ätznatron in 15 $cm^3$ Wasser. Nach 15 Minuten Erhitzen am Rückfluß löst sich das Pyron völlig, und anschließend wird 1 Stunde weitererhitzt. Durch Kühlen erstarrt der Kolbeninhalt, und man filtriert durch eine Glasfritte. Das Filtrat (IV$\beta$), (welches das Hydroxysalz des Pyrons $\beta$ enthält), wird zurückbehalten und der Niederschlag (IV$\alpha$) (Hydroxysalz des Pyrons $\alpha$) aus Wasser umkristallisiert. Schmelzpunkt = 274°C. Das Pyron (III$\alpha$) erhält man dadurch, daß man das umkristallisierte Hydroxysalz (IV$\alpha$) mit 5 $cm^3$ 10%iger wäßriger Salzsäure 1 Stunde am Rückfluß erhitzt. Nach Abkühlung extrahiert man mit Methylenchlorid. Die organische Phase wird mit Natriumbicarbonat und danach mit Wasser neutral gewaschen. Nach Trocknung über Natriumsulfat wird das Lösungsmittel abgedampft. Das gewonnene rohe Pyron (0,300 g; Ausbeute: 30%) kristallisiert man aus absolutem Alkohol um. Man erhält 0,270 g Pyron (III$\alpha$); Schmelzpunkt = 136—137° C, Ausbeute: 27%.

Das Pyron (III$\beta$) erhält man durch Ansäuern des Filtrats (IV$\beta$) mit 10%iger Salzsäurelösung und Rühren bei Raumtemperatur für eine Stunde und 15 Minuten. Man extrahiert mit Methylenchlorid, und die organische Phase wird mit Natriumbicarbonat und danach mit Wasser neutral gewaschen. Nach Trocknung über Natriumsulfat wird das Methylenchlorid abgedampft. Das gewonnene rohe Pyron (0,600 g, Ausbeute: 60%) kristallisiert man aus absolutem Alkohol um. Man erhält 0,520 g Pyron (III$\beta$), Schmelzpunkt = 159—160° C; Ausbeute: 52%.

## Beispiel VI

### Herstellung von cis-4-(4'-Chlorphenyl)-6-methyl-3-phenyl-3,4,5,6-Tetrahydropyron-2 III

a) Herstellung von erythro-2-(4'-Chlorphenyl)-4-oxo-1-phenylpentancarbonitril

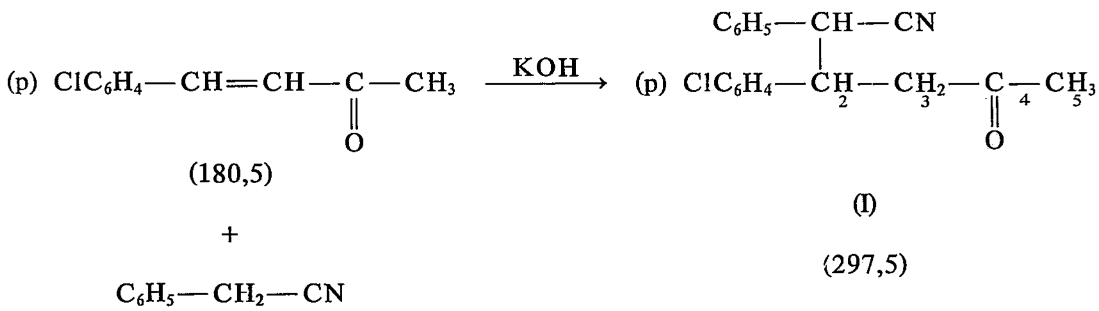


(180,5)

\+

(117)

(I)

(297,5)

Zur Herstellung dieses Produkts wendet man die in Beispiel I beschriebene Methode an.

b) Herstellung der erythro-3-(4'-Chlorphenyl)-5-oxo-2-phenylhexansäure

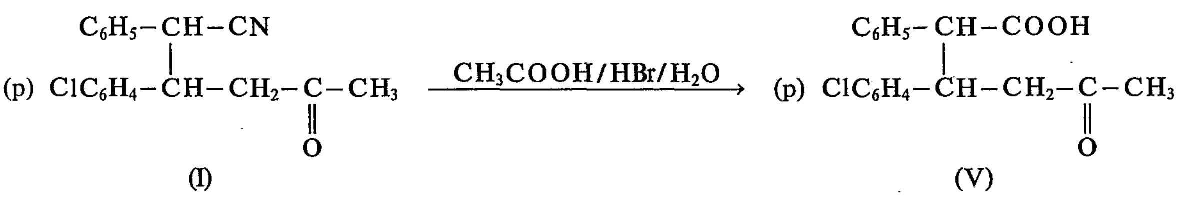


(I)

(297,5)

(V)

(316,5)

Man erhitzt 60 g (0,2 Mol) des Ketonitrils (I) 5 Stunden am Rückfluß in 350 $cm^3$ einer Wasser/Essigsäure/Bromwasserstoffsäurelösung, die man aus 700 $cm^3$ Essigsäure, 166 $cm^3$ azeotropischer Bromwasserstoffsäure und 44 $cm^3$ Wasser erhielt. Nach Abkühlung gießt man die Lösung in 1000 $cm^3$ Eiswasser. Die Säure scheidet sich zunächst als Öl aus, welches anschließend kristallisiert. Die Kristalle werden abfiltriert und mit Wasser neutral gewaschen, um die Essigsäure zu entfernen. Man gewinnt 52 g Rohprodukt. Ausbeute: 82%. Schmelzpunkt: 203—208° C.

Man kristallisiert aus absolutem Alkohol um. Schmelzpunkt: 204—205° C; Ausbeute: 80%.

IR (KBr): $\nu$ O—H: 3500—2500 $cm^{-1}$, $\nu$ C=O: 1695—1710 $cm^{-1}$

Analyse: ($C_{18}H_{17}ClO_3$)

| | | | |
|---|---|---|---|
| Berechnet % | C 68,35 | H 5,37 | Cl 11,08 |
| Gefunden % | C 68,45 | H 5,42 | Cl 11,01 |

Am Rohprodukt (VII) durchgeführte NMR zeigt die Gegenwart von 2 Diastereoisomeren (bezüglich der Kohlenstoffatome 2 und 3). Die am Signal der Protonen $H_{(6)}$ durchgeführte Messung ergibt den Prozentsatz für jedes Diastereoisomer:

- erythro: 75%
- threo: 25%.

Umkristallisation aus absolutem Äthanol ermöglicht es, das erythro-Isomer rein zu erhalten.

NMR: $\delta$: 7,30–7,60 ppm, Multiplett für 9 aromatische Protonen.
($CD_3COCD_3$) + (DMSO)-$d_6$
$\delta$: 3,90 ppm, Multiplett für 2 Protonen $H_{(2)}$; $H_{(3)}$.
$\delta$: 2,60 ppm, Multiplett für 2 Protonen $H_{(4)}$.
$\delta$: 1,80 ppm, Singulett für 3 Protonen $CH_{3(6)}$.

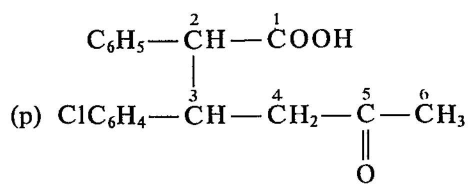
(V)

c) Herstellung der erythro-3-(4′-Chlorphenyl)-5-hydroxy-2-phenylhexansäure

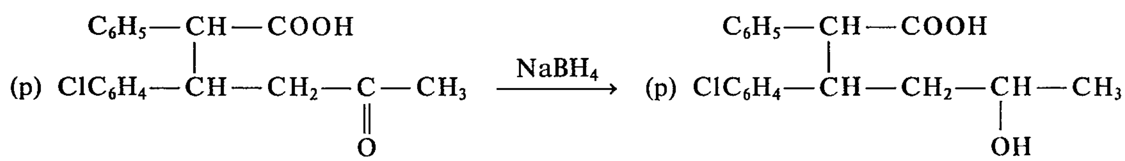


(V) (VI)

(316,5) (318,5)

40 g (0,12 Mol) der Säure (V) (reines erythro-Diastereoisomer) werden in 400 cm³ Wasser suspendiert. Man löst 10 g $NaBH_4$ (10mal die theoretische Menge) in 200 cm³ Wasser unter Zusatz von 50 cm³ 2n-Natronlauge. Die $NaBH_4$-Lösung gibt man langsam bei Raumtemperatur zur Suspension der Säure. Nach der Zugabe wird 12 Stunden weitergerührt. Dann säuert man das Medium mit der erforderlichen Menge konzentrierter Salzsäure an.

Die gebildete Hydroxysäure wird mit 200, 100, 50 und 60 cm³ eines Methylenchlorid/Äthergemischs (50/50) extrahiert. Die gewonnenen organischen Phasen werden mit Natriumcarbonat neutral und dann mit Wasser gewaschen. Die organische Phase trocknet man über Natriumsulfat. Nach Abdampfen der Lösungsmittel gewinnt man 32 g Rohprodukt. Ausbeute: 80%. Schmelzpunkt = 80° C.

Die erythro-Hydroxysäure (VI) läßt sich nicht durch Umkristallisation reinigen, denn beim Erhitzen ihrer Lösungen wird sie zum Pyron zyklisiert.

IR (KBr): $\nu$ O—H (Alkohol): 3400 cm⁻¹; $\nu$ OH (Säure): 3500–2500 cm⁻¹.
$\nu$ C=O: 1700 cm⁻¹.

Massenspektrum: $C_{18}H_{19}O_3Cl$ (318)
Gefunden: 300, 302 (3 : 1) $M^+ - 18$
165, 167 (3 : 1) (p) $ClC_6H_4-CH=CH-CH^+-CH_3$
118 (3 : 1) $C_6H_5-CH-C{\equiv}O^+$

d) Herstellung von cis-4-(4'-Chlorphenyl)-6-methyl-3-phenyl-3,4,5,6-tetrahydropyron-2

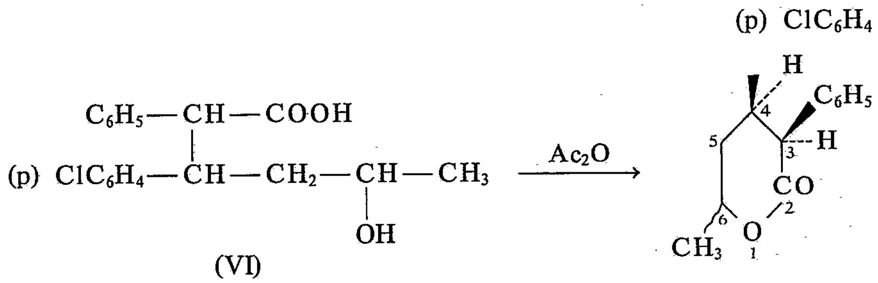


(VI)

(318,5)

(III)

(300,5)

Man erhitzt 30 g der Hydroxysäure (VI) (0,09 Mol) in 150 $cm^3$ Essigsäureanhydrid eine Stunde am Rückfluß.

Nach Destillation des Essigsäureanhydrids bei vermindertem Druck nimmt man den Rückstand in 200 $cm^3$ Chloroform auf. Man wäscht mit Wasser neutral. Die organische Phase wird über Natriumsulfat getrocknet. Nach Abdampfen des Lösungsmittels gewinnt man 21 g Rohprodukt. Ausbeute: 75%. Schmelzpunkt = 180—185° C.

IR (KBr): $\nu$ C=O: 1725 $cm^{-1}$.

Massenspektrum: $C_{18}H_{17}ClO_2$ : 300
Gefunden: 300, 302 (3 : 1): $M^+$
165, 167 (3 : 1): (p)$ClC_6H_4-CH=CH-CH^+-CH_3$
118: $C_6H_5-CH-C\equiv O^+$

NMR: ($CD_3COCD_3$ + DMSO)
$\delta$: 6,7—7,6 ppm, Multiplett für 9 aromatische Protonen.
$\delta$: 4,7 ppm, Multiplett für 1 Proton $H_{(6)}$.
$\delta$: 4,2 ppm, Dublett für 1 Proton $H_{(3)}$.
J $H_{(3)}$, $H_{(4)}$: 6,5 Hz.
$\delta$: 3,7 ppm, Multiplett für 1 Proton $H_{(4)}$.
$\delta$: 1,9 ppm, Multiplett für 2 Protonen $H_{(5)}$ der beiden Isomeren.
$\delta$: 1,3 ppm, Dublett für 3 Protonen des $-CH_{3(6)}$ für ein Diastereoisomer. J $CH_3$, $H_{(6)}$ = 6 Hz.
$\delta$: 1,4 ppm, Dublett für 3 Protonen $CH_{3(6)}$ für das zweite Diastereoisomer. J $CH_3$, $H_{(6)}$ = 6 Hz.

Die Kupplungskonstante von 6,5 Hz zwischen den Protonen (3) und (4) zeigt, daß das erhaltene Pyron die cis-Struktur besitzt.

Weiterhin ergibt die Bestimmung der beiden, bei der Reduktion der Carbonylfunktion in der Ketosäure (V) erhaltenen Diastereoisomeren deren Prozentanteil.

a/$\beta$ : 60/40 (Rohprodukt).

Umkristallisation aus Alkohol ermöglicht es, ein Isomer des erhaltenen Pyrons anzureichern. Die NMR-Bestimmung zeigt dann eine $\alpha/\beta$-Zusammensetzung von 80/20. Schmelzpunkt = 182—185° C.

Eine zweite Umkristallisation aus Äthanol liefert ein Produkt mit einem Schmelzpunkt = 186—190° C. NMR zeigt einen Prozentgehalt an $\alpha$ über 90%. Nach Durchführung einer dritten Umkristallisation zeigt das bei 250 MHz gemessene Produkt einen Gehalt des $\alpha$-Diastereoisomeren, der praktisch gleich 100% ist.

## Beispiel VII

Herstellung von 4-(4'-Chlorphenyl)-6-methyl-3-phenyl-3,4,5,6-tetrahydropyron-2 über 3-(4'-Chlorphenyl)-5-oxo-2-phenylhexansäure

a) Herstellung von erythro-2-(4'-Chlorphenyl)-4-oxo-1-phenylpentancarbonitril

Zur Synthese des Produktes I wendet man die in Beispiel I beschriebene Methode an.

NMR zeigt, daß das erhaltene Produkt nach Umkristallisation aus absolutem Äthanol 90%

erythro-Isomer und 10% threo-Isomer enthält. Schmelzpunkt: 130—132° C.
Ausbeute der Umsetzung: 90%.
Ausbeute der Umkristallisation: 90%.

b) Herstellung der erythro-3-(4'-Chlorphenyl)-5-oxo-2-phenylhexansäure

Zur Herstellung des Produkts (V) wendet man die in Beispiel VI beschriebene Methode an.

Nach zwei Umkristallisationen aus absolutem Alkohol zeigt NMR ausschließlich die Gegenwart der erythro-Ketosäure. Schmelzpunkt = 204—205° C.
Ausbeute der Umsetzung: 80%
Ausbeute der Umkristallisation: 80%.

c) Herstellung des Natriumsalzes der
erythro-3-(4'-Chlorphenyl)-5-oxo-2-phenylhexansäure

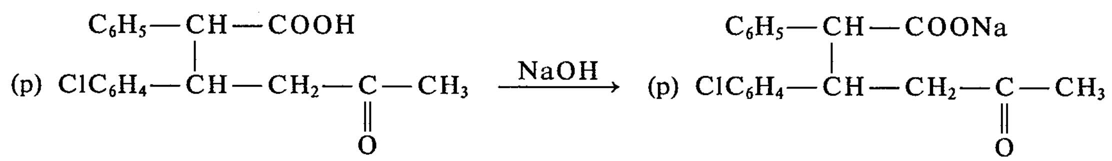


(V) (VII)

(316,5) (338,5)

Man suspendiert 17 g (0,054 Mol) dieser Säure (V) in 150 $cm^3$ absolutem Alkohol. Das Produkt löst sich bei leichtem Erhitzen auf 40° C. Man versetzt langsam mit Natronlauge (10% Überschuß). Nach beendeter Zugabe fällt das gebildete Salz aus. Das Äthanol wird abgedampft und das erhaltene Produkt aus Alkohol/Wassergemisch (80 : 20) umkristallisiert. Man gewinnt 17 g Produkt. Ausbeute: 90%; Schmelzpunkt = 283° C.

IR (KBr): $\nu$ C=O: 1705 $cm^{-1}$; $\nu$ $COO^-$: 1580 und 1390 $cm^{-1}$.

NMR ($D_2O$:

$\delta$: 7,3 ppm, Multiplett für 9 aromatische Protonen.
$\delta$: 3,65 ppm, Multiplett für 2 Protonen. $H_{(2)}$, $H_{(3)}$.
$\delta$: 2,50 ppm, Multiplett für 2 Protonen, $H_{(4)}$.
$\delta$: 1,70 ppm, Singulett für 3 Protonen $CH_{3(6)}$.

Dieses Ketosalz säuert man mit Salzsäure an. Die dabei erhaltene entsprechende Säure wird zur Bestimmung der Konfiguration des gebildeten Ketosalzes verwendet.

Die mit der Säure durchgeführte NMR führt dazu, der Säure und infolgedessen dem entsprechenden Salz die erythro-Konfiguration zuzuschreiben.

d) Herstellung von cis- + trans-4-(4′-Chlorphenyl)-6-methyl-3-phenyl-3,4,5,6-tetrahydropyron-2 III

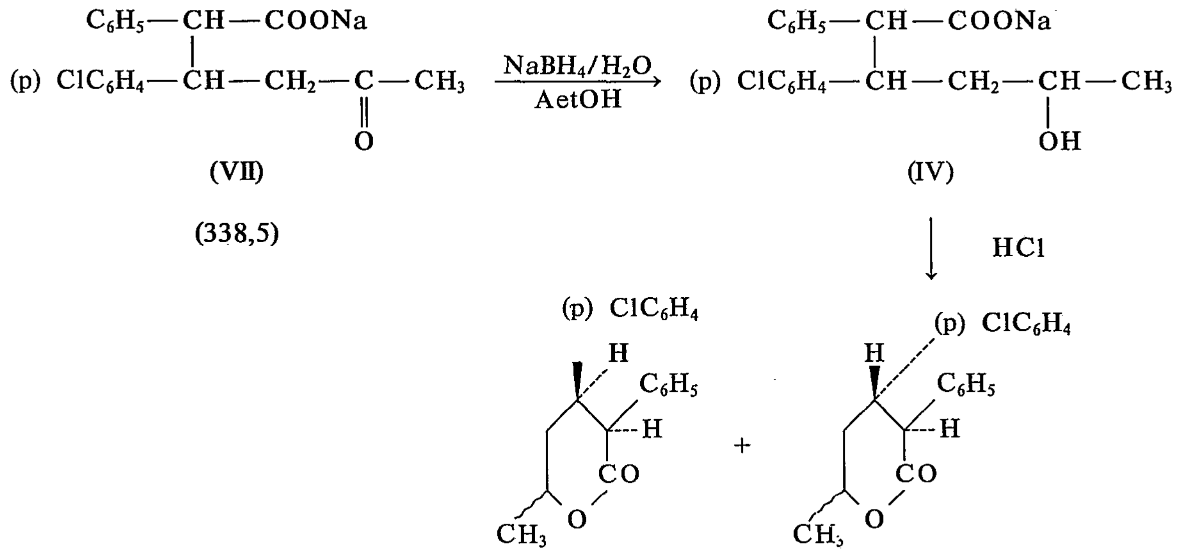


(III) cis (III) trans

Man reduziert 10 g Ketosalz (VI) (0,03 Mol) mit 1 g $NaBH_4$ in 100 cm³ absolutem Alkohol. Nach 12 Stunden Rühren bei Raumtemperatur wird der Alkohol abgedampft.

Durch eine Stunde Erhitzen des rohen Hydroxysalzes (IV) am Rückfluß mit einer Lösung von 5 cm³ Salzsäure in 100 cm³ Wasser erhält man das Pyron. Nach Abkühlung wird mit Chloroform extrahiert. Die organische Phase wird mit Wasser neutral gewaschen. Nach Trocknung über Natriumsulfat dampft man das Chloroform ab. Man gewinnt 5,3 g Rohprodukt. Ausbeute: 60%.

## Beispiel VIII

Herstellung des reinen cis-Pyrons aus dem erythro-Ketosalz

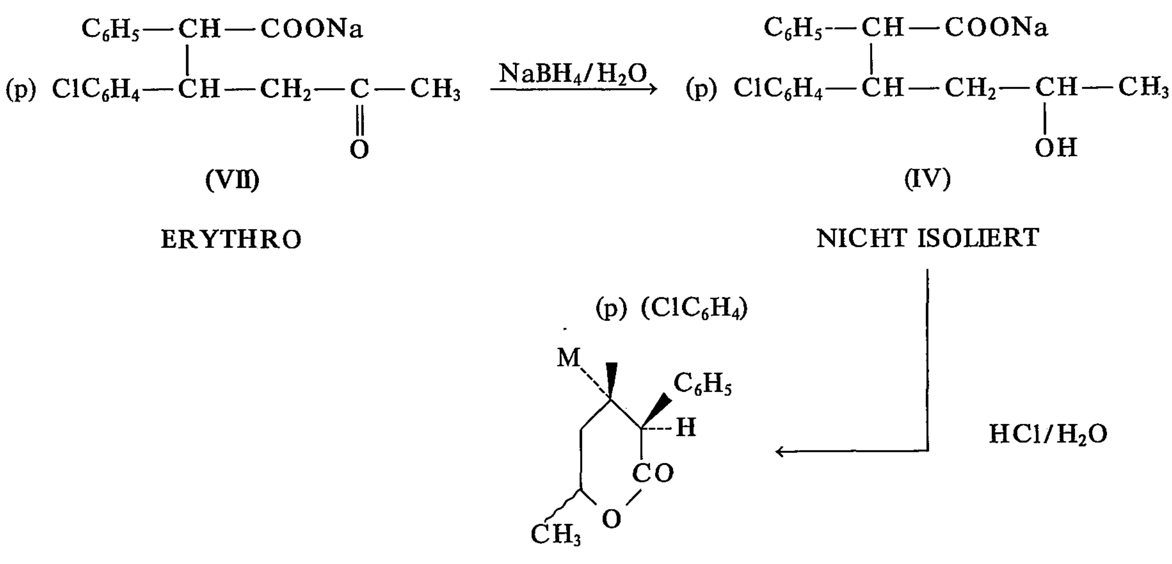


(III) cis

Man suspendiert 2 g Ketosalz (VII) in 20 cm³ Wasser. 200 mg $NaBH_4$ in 5 cm³ Wasser mit Zusatz einiger Tropfen 2n-Natronlauge werden langsam dazugegeben. Das Reaktionsgemisch wird 12 Stunden bei Raumtemperatur weitergerührt.

Nach 12 Stunden säuert man mit konzentrierter Salzsäure an und erhitzt 1 Stunde am Rückfluß. Nach Abkühlung extrahiert man das gebildete Pyron mit Chloroform, wäscht mit Wasser neutral und trocknet.

Nach Abdampfen des Lösungsmittels gewinnt man 1 g Rohprodukt. Ausbeute: 60%.

Die mit dem Rohprodukt durchgeführte NMR zeigt, daß ausschließlich das cis-Pyron vorliegt. Man beobachtet also keine Epimerisierung, wenn die Reduktion in völlig wäßrigem Medium durchgeführt wird. Dies verdeutlicht die epimerisierende Rolle des Reduktionssystems $NaBH_4$/AetOH.

## Beispiel IX

### Herstellung von trans- und cis-4-(4'-Chlorphenyl)-6-methyl-3-phenyl-3,4,5,6-tetrahydropyron-2 IIIα

#### a) Herstellung von erythro-3-(4'-Chlorphenyl)-5-oxo-2-phenylhexansäureäthylester

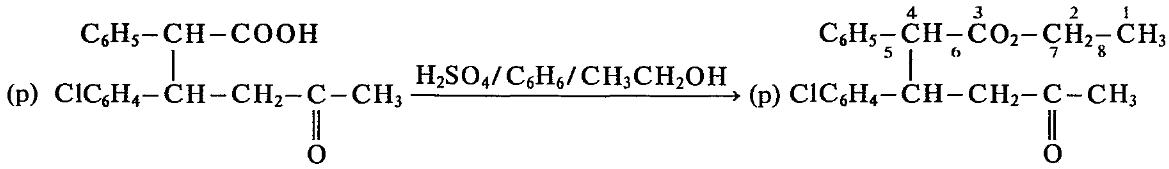


(V) (VIII)

(316,5) (344,5)

Die Herstellung des reinen, zur Bereitung des Esters verwendeten erythro-Isomeren der Säure (V) ist in Beispiel VI beschrieben.

Man erhitzt 10 g der Säure (V) (0,03 Mol) in einem Gemisch aus Benzol (100 cm³) und Äthanol (60 cm³) unter Zusatz von 10 cm³ konzentrierter Schwefelsäure 6 Stunden am Rückfluß. Das während der Veresterung freigesetzte Wasser wird durch azeotropische Destillation aus dem Reaktionsgemisch entfernt. Nach Abkühlung werden das überschüssige Äthanol und das Benzol abgedampft. Der Rückstand wird in Chloroform aufgenommen und die organische Phase mit Bicarbonat und mit Wasser neutral gewaschen. Die organische Phase trocknet man über Natriumsulfat und dampft das Lösungsmittel ab. Man gewinnt 9 g Produkt.

Die mit dem gebildeten Esterrohprodukt durchgeführte NMR zeigt die Gegenwart eines einzigen Diastereoisomeren bezüglich der Kohlenstoffatome (4) und (5). Unter der Annahme, daß die Veresterung der erythro-Säure (V) keine Epimerisierung hervorruft, besteht der erhaltene Ester also ausschließlich aus der erythro-Form.

Schmelzpunkt: 95—97° C. Ausbeute: 90%.

Das Produkt wird aus absolutem Äthanol umkristallisiert.

Schmelzpunkt: 105—106° C. Ausbeute: 80%.

#### b) Herstellung des erythro-3-(4'-Chlorphenyl)-5-hydroxy-2-phenylhexansäureäthylesters

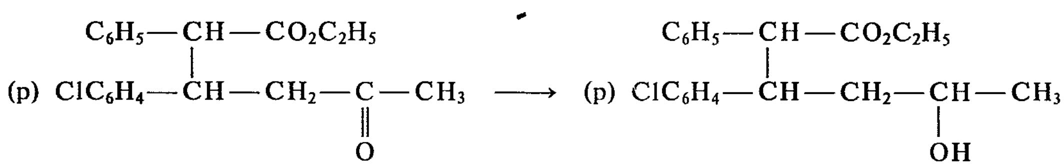

(VIII) (IX)

(344,5) (346,5)

Man suspendiert 10 g Ketoester (VIII) in 100 cm³ absolutem Äthanol. Die Estersuspension versetzt man langsam mit 1 g $NaBH_4$ in 10 cm³ Wasser mit Zusatz eines halben Plätzchens Ätznatron. Man rührt das Reaktionsgemisch 3 Stunden bei 15°C. Dann wird das Äthanol abgedampft und der Rückstand in Chloroform aufgenommen. Die organische Phase wird mit Wasser neutral gewaschen. An dem Rohprodukt durchgeführte NMR zeigt die Gegenwart eines einzigen Diastereoisomeren bezüglich der Kohlenstoffatome 4 und 5. Die Konfiguration wird ferner als erythro bestimmt. Die zwei neuen, durch Reduktion des Carbonyls entstandenen Diastereoisomeren bezüglich des Kohlenstoffatoms 7 sind in NMR nicht sichtbar, vermutlich wegen freier Rotation um die Kohlenstoff-Kohlenstoffbindung. Das Produkt wird aus einem Tetrachlorkohlenstoff/Hexangemisch umkristallisiert. Schmelzpunkt = 104—105° C. Ausbeute: 52%

IR (KBr): $\nu$ O—H: 3380 cm$^{-1}$, $\nu$ C=O (Ester): 1725 cm$^{-1}$

NMR ($CD_3COCD_3$)

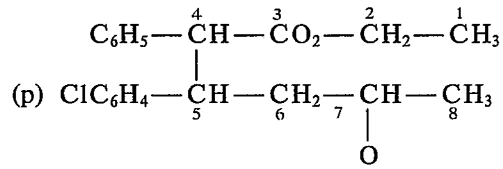

(IX)

δ: 7,40 ppm, Multiplett für 9 aromatische Protonen.
δ: 3,80 ppm, Multiplett für 5 Protonen $H_{(2)}$, $H_{(4)}$, $H_{(5)}$ und $H_{(7)}$.
δ: 3,30 ppm, Signal für 1 Proton: O—H.
δ: 1,50 ppm, Multiplett für 2 Protonen: $H_{(6)}$.
δ: 0,95 ppm, Dublett für 3 Protonen: $CH_{3(8)}$;
J $H_{(8)}$, $H_{(7)}$ = 6,0 Hz.
δ: 0,90 ppm, Triplett für 3 Protonen: $CH_{3(1)}$;
J $H_{(1)}$, $H_{(2)}$ = 6,5 Hz.

Analyse: $C_{20}H_{23}ClO_3$

| | | | |
|---|---|---|---|
| Berechnet % | C 69,26 | H 6,68 | Cl 10,22 |
| Gefunden % | C 69,34 | H 6,50 | Cl 10,32 |

c) Herstellung des Natriumsalzes der 3-(4'-Chlorphenyl)-5-hydroxy-2-phenylhexansäure

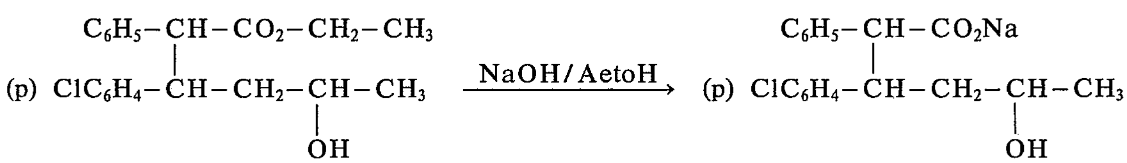


(IX) (IV)

(346,5) (340,5)

Man löst 5 g des reinen erythro-Isomeren des Hydroxyesters (IX) in der Wärme in 50 cm³ Äthanol und versetzt langsam mit Ätznatron (10% Überschuß) in 10 cm³ Wasser. Nach 15 Stunden Erhitzen am Rückfluß wird das Reaktonsgemisch abgekühlt und das Äthanol zum Teil abgedampft. Ein Produkt kristallisiert, und dieses wird in der Wärme wieder aufgelöst und heiß filtriert. Ein gelbliches Produkt erstarrt. Nach Filtrieren kristallisiert man dieses aus Alkohol/Wassergemisch (50/50) um. Man gewinnt 2,5 g umkristallisiertes Produkt. Ausbeute = 50%. Schmelzpunkt = 273—274°C. IR bestätigt die Bildung des Hydroxysalzes.

d) Herstellung von trans-4-(4'-Chlorphenyl)-6-methyl-3-phenyl-3,4,5,6-tetrahydropyron-2 IIIα

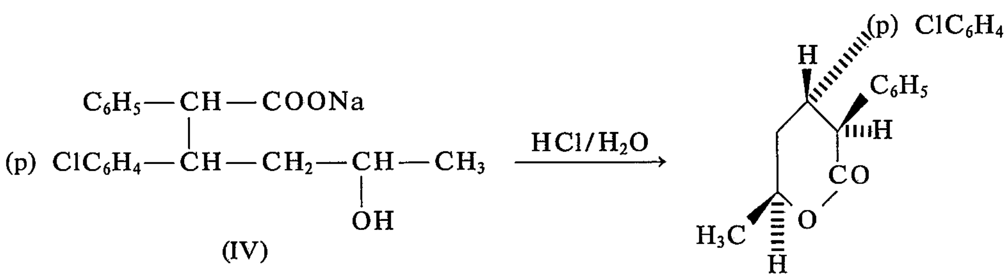


(IV) trans (IIIα)

(340,5) (300,5)

Man erhält das Pyron (IIIα) durch eine Stunde Erhitzen des umkristallisierten Hydroxysalzes (IV) mit einer Lösung von 1 cm³ konzentrierter Salzsäure in 20 cm³ Wasser am Rückfluß. Nach Abkühlung extrahiert man das Pyron mit Chloroform. die organische Phase wird mit Bicarbonat und mit Wasser neutral gewaschen.

Nach Trocknung über Natriumsulfat wird das Chloroform abgedampft. Mit dem rohen Pyron durchgeführte NMR zeigt die Gegenwart eines einzigen Diastereoisomeren bezüglich des Kohlenstoffatoms 6. Aus einem Vergleich mit den in Beispiel I erhaltenen Ergebnissen läßt sich herleiten, daß es sich dabei um das Pyron (IIIα) handelt.

Diese Methode liefert das α-Diastereoisomer direkt auf synthetischem Weg und nicht mehr durch fraktionierte Umkristallisationen.

Ausbeute: 45%; Schmelzpunkt = 128—131° C.

Das erhaltene Produkt wird aus Äthanol umkristallisiert. Schmelzpunkt: 136—137° C.

NMR ($CD_3COCD_3$)

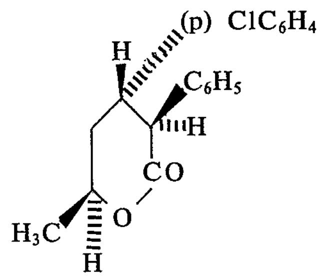

(IIIα) trans

δ: 7,10 ppm, Signal für 9 aromatische Protonen.
δ: 4,95 ppm, Multiplett für 1 Proton: $H_{(6)}$.
δ: 4,15 ppm, Dublett für 1 Proton: $H_{(3)}$.
$J\ H_{(3)}, H_{(4)} = 11{,}5$ Hz.
δ: 3,60 ppm, Multiplett für 1 Proton: $H_{(4)}$.
δ: 2,20 ppm, Multiplett für 2 Protonen: $H_{(5)}$.
δ: 1,42 ppm, Dublett für 3 Protonen:
$CH_{3(6)}$. $J\ CH_3, H_{(6)} = 6$ Hz.

Die Gegenwart eines einzigen Dubletts bei 4,15 ppm zeigt, daß tatsächlich nur ein einziges Diastereoisomer bezüglich $C_6$ vorliegt.

e) Herstellung von cis-4-(4'-Chlorphenyl)-6-methyl-3-phenyl-3,4,5,6-tetrahydropyron-2 IIIα

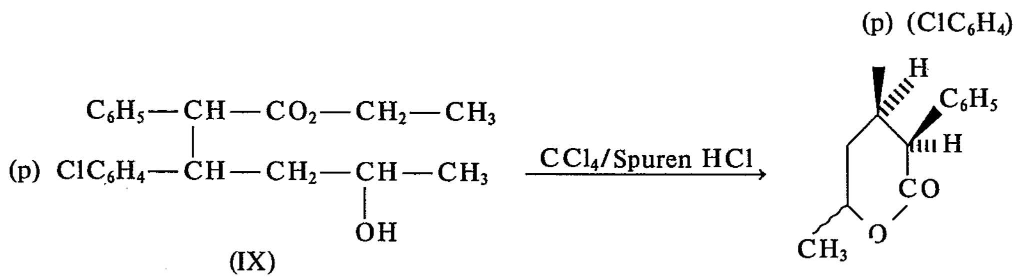


(IX)

(346,5)

(IIIα) cis

Man erhitzt 2 g Hydroxyester (IX) eine Stunde am Rückfluß mit 50 cm³ $CCl_4$ und 1 cm³ 0,1n-Salzsäure. Die Lösung wird abgekühlt. Dabei bildet sich ein kristallisiertes Produkt, das man durch eine Glasfritte abfiltriert.

Mit dem Produkt durchgeführte NMR zeigt die Gegenwart eines cis-Pyrons, das sehr reich an einem Isomeren bezüglich des Kohlenstoffatoms 6 ist. Genaue Messung ergibt a/β = 90/10. (α und β sind in der Tat die beiden Diastereoisomeren bezüglich des asymmetrischen Kohlenstoffatoms $C_6$). Schmelzpunkt = 186—190° C. Man führt eine Umkristallisation aus Alkohol durch. Das erhaltene umkristallisierte Produkt wird durch NMR bei 80 MHz vermessen. NMR zeigt nur die Gegenwart von 100% eines einzigen Diastereoisomeren. Schmelzpunkt = 189—190° C.

NMR (80 MHz): ($CD_3COCD_3$ + DMSO-$d_6$

δ: 7,40 ppm, Signal für 5 aromatische Protonen (Phenyl an $C_3$).

δ: 6,90 ppm, Signal für 4 aromatische Protonen (Phenyl an $C_4$).
δ: 4,90 ppm, Multiplett für 1 Proton : $H_{(6)}$.
δ: 4,25 ppm, Dublett für 1 Proton : $H_{(3)}$
$J\ H_{(3)}, H_{(4)} = 6$ Hz.
δ: 3,50 ppm, Multiplett für 1 Proton : $H_{(4)}$.
δ: 2,10 ppm, Multiplett für 2 Protonen : $H_{(5)}$.
δ: 1,55 ppm, Dublett für 3 Protonen : $CH_{3(6)}$.
$J\ CH_3, H_{(6)} = 6$ Hz.

Dieses Verfahren gestattet es somit, die beiden isomeren cis- und trans-Pyrone und, was noch viel wichtiger ist, selektiv ein einziges Diastereoisomer jeden Pyrons, das α-Diastereoisomer, aus demselben Produkt, dem Hydroxyester (IX), zu erhalten.

Bericht über die zur Demonstration der therapeutischen Eigenschaften der Verbindungen der allgemeinen Formeln IIIa und IIIb durchgeführten pharmakologischen Versuche

Die erfindungsgemäßen halogenierten δ-Hydroxycarbonsäurederivate besitzen bemerkenswerte therapeutische Eigenschaften und insbesondere eine psychostimulierende antidepressive Wirkung, wie aus den unter Verwendung dieser Derivate durchgeführten Versuchen ersichtlich ist.

Sämtliche Versuche, über die unten berichtet wird, wurden an männlichen Ratten mit einem Gewicht zwischen 170 und 220 g (Stamm Wistar AF) und an männlichen Mäusen mit einem Gewicht zwischen 18 und 23 g (Stamm Swiss NMRI) ausgeführt, falls im folgenden nicht anders angegeben.

Alle Versuche wurden in einem Laboratorium mit konstant gehaltener Temperatur (22±1°C) durchgeführt.

Sämtliche Versuche erfolgten blind (während der Durchführung der Prüfungen war es dem Experimentator nicht bekannt, welche Tiere den mutmaßlich aktiven Stoff in welcher Dosis erhalten hatten).

Falls nicht anders angegeben wurden stets Gruppen von je 10 Tieren verwendet.

Bestimmung der akuten Toxizität an der Maus

Angewandte Methode: Behrens und Karber

| Substanz | $LD_{50}$ (orale Verabreichung) | $LD_{50}$ (intraperitoneale Verabreichung) |
|---|---|---|
| 4-(4'-Chlorphenyl)-6-methyl-3-phenyl-3,4,5,6-tetrahydropyron-2 (Beispiel I) | 1050 mg/kg | 520 mg/kg |
| $(p)ClC_6H_4-CH-CH_2-CHOH-CH_3$<br>$C_6H_5-CH-CO_2Na$<br>(Produkt IV, gemäß Beispiel IX erhalten) | 310 mg/kg | 160 mg/kg |

Beobachtungen an den Tieren

Sowohl an der Ratte wie an der Maus beobachtet man bei unter den toxischen liegenden Dosierungen eine leichte Erregung mit erhöhter Reaktivität auf Berührung oder auf Schallstimuli. Diese Stimulierung ist von keiner motorischen Inkoordination oder anormalen oder stereotypen Bewegungen begleitet. Ferner stellt man unter diesen Bedingungen keine Änderung des Pupillendurchmessers fest, jedoch eine deutliche Hyperthermie. Die stereotypen Bewegungen sind ab 4 bis 8 mg · $kg^{-1}$ bei dem gemäß Beispiel I erhaltenen Produkt und ab 8 bis 32 mg · $kg^{-1}$ bei dem gemäß Beispiel IX erhaltenen Produkt IV zu beobachten.

Nur bei der Letaldosis sehr naheliegenden Dosierungen beobachtet man Zittern, sehr starke Hyperreaktivität auf Stimuli, gegenseitige Angriffslust und Krampferscheinungen unter Ausstrecken der Hinterpfoten. Unter diesen Bedingungen zeigt sich ferner eine Hyperthermie von ungefähr 1°C.

Außerdem beobachtet man an der Ratte ein verstärktes sexuelles Betragen.

Motilität der Maus

Die Motilität wird mit Hilfe von photoelektrischen Zellenaktimetern bestimmt (Boissier und Simon, Arch. int. Pharmacodyn. 1965, 158, 212—221). Man verwendet stets mindestens 10 Tiere pro Gruppe.

Kurz nach der oralen oder intraperitonealen Verabreichung des erfindungsgemäßen, nach Beispiel I erhaltenen Produkts werden die Mäuse in das Aktimeter eingeführt. Die Motilität wird von der Einführung in das Aktimeter bis zur 60. Minute gemessen. Die Zähler werden nach 30 Minuten abgelesen, dann auf 0 zurückgestellt und für 30 Minuten alle 10 Minuten abgelesen.

Die erhaltenen Ergebnisse sind in den nachfolgenden Tabellen Ia und Ib angeführt:

Tabelle Ia

Einfluß des gemäß Beispiel I erhaltenen Produkts auf die Motilität der Maus

| Anzahl Mäuse | mg/kg | Verabreichungsweg | Anzahl von den Mäusen durchlaufener Strahlen nach : (Minuten) | | | | | | | |
|---|---|---|---|---|---|---|---|---|---|---|
| | | | 30 | Sa | Test | 30+10 | 30+20 | 30+30 | Sa | Test |
| 40 | 0 | intraperitoneal | 383 | 18 | | 56 | 120 | 181 | 21 | |
| 10 | 1 | intraperitoneal | 400 | 32 | NS | 76 | 163 | 235 | 42 | NS |
| 20 | 2 | intraperitoneal | 474 | 33 b) | 2684 | 114 | 231 | 331 | 33 c) | 4042 |
| 10 | 4 | intraperitoneal | 529 | 17 c) | 4005 | 129 | 269 | 322 | 50 b) | 2951 |
| 10 | 8 | intraperitoneal | 659 | 66 c) | 5827 | 147 | 309 | 467 | 85 c) | 4945 |
| 20 | 16 | intraperitoneal | 763 | 50 c) | 8809 | 176 | 336 | 465 | 56 c) | 5859 |
| 10 | 32 | intraperitoneal | 891 | 77 c) | 9967 | 187 | 422 | 594 | 102 | 6481 |
| 30 | 0 | oral | 507 | 25 | | 116 | 236 | 321 | 26 | |
| 20 | 4 | oral | 462 | 30 | NS | 139 | 257 | 386 | 37 | NS |
| 20 | 8 | oral | 478 | 32 | NS | 157 | 314 | 461 | 41 b) | 3096 |
| 20 | 16 | oral | 629 | 27 c) | 3298 | 232 | 428 | 636 | 31 c) | 7902 |
| 10 | 32 | oral | 783 | 72 c) | 4660 | 270 | 587 | 869 | 62 | 9377 |

a) = Differenz signifikant auf der Stufe 0,05.
b) = Differenz signifikant auf der Stufe 0,01.
c) = Differenz signifikant auf der Stufe 0,001.

Sa = Standardabweichung.
NS = nicht signifikant.

Tabelle Ib

Einfluß des gemäß Beispiel I erhaltenen Produkts auf die Motilität der Maus

| Anzahl Mäuse | mg/kg | Verabreichungsweg | Anzahl von den Mäusen durchlaufener Strahlen nach : (Minuten) | | | | | | | |
|---|---|---|---|---|---|---|---|---|---|---|
| | | | 30 | Sa | Test | 30+10 | 30+20 | 30+30 | Sa | Test |
| 10 | 0 | oral | 476 | 29 | | 105 | 206 | 271 | 39 | |
| 10 | 2 | oral | 417 | 28 | | 119 | 221 | 334 | 40 | |
| 10 | 4 | oral | 472 | 27 | | 129 | 259 | 376 | 43 | NS |
| 10 | 8 | oral | 452 | 37 | | 141 | 286 | 443 | 48 | 2,770 b) |
| 10 | 16 | oral | 565 | 35 | NS | 172 | 359 | 537 | 47 | 4,35 c) |
| 10 | 32 | oral | 560 | 35 | NS | 184 | 376 | 547 | 60 | 3,830 c) |

b) = Differenz signifikant auf der Stufe 0,01.
c) = Differenz signifikant auf der Stufe 0,001.
NS = nicht signifikant.

Untersuchung von Stereotypien an der Ratte:

Die Versuche werden mit Gruppen von je 6 Ratten je Dosis durchgeführt; sofort nach der Verabreichung auf oralem oder intraperitonealem Wege bringt man die Tiere in einzelne, 20 × 10 × 10 cm große durchsichtige Kunststoffkästen, die mit einem Deckel aus durchlochtem rostfreien Stahl versehen sind. Die stereotypen Bewegungen werden alle 10 Minuten nach der von Simon und Chermat (J. Pharmacol. [Paris], 1972, 3, 235—238) beschriebenen Methode mit 0 bis 3 bewertet, bis sie bei allen Tieren verschwunden sind. Die Kurven werden im einzelnen aufgetragen und die Fläche darunter wie im obigen Zitat beschrieben berechnet.

Die als mittlere Fläche unter den Kurven ausgedrückten Ergebnisse sind in der beigefügten Abbildung angegeben. Sie zeigen das Vorliegen sehr erheblicher Stereotypien ab 8 mg · $kg^{-1}$. Wie aus der Abbildung ersichtlich ist die Aktivität des gemäß Beispiel II erhaltenen Produkts bei oraler Verabreichung etwas höher.

Antireserpinwirkung an der Maus:

Zur Zeit 0 erhalten die Mäuse auf intraperitonealem Wege Reserpin (2,5 mg · $kg^{-1}$.

4 Stunden nachdem sich eine erhebliche Hypothermie eingestellt hat (30 bis 31° C) verabreicht man die zu prüfende Substanz auf intraperitonealem oder oralem Wege. Mit Hilfe einer Sonde, die ein auf konstante Tiefe eingeschobenes Thermoelement trägt, mißt man 2 Stunden lang alle 30 Minuten die Rektaltemperatur.

Die Ergebnisse sind im einzelnen in der nachfolgenden Tabelle IIa und IIb angeführt:

Tabelle IIa

Antireserpinwirkung des gemäß Beispiel I erhaltenen Produkts

| Anzahl Mäuse | mg/kg | Verabreichungsweg | Rektaltemperaturen °C ± SA nach : (Minuten) 30 | 60 | 90 | 120 |
|---|---|---|---|---|---|---|
| 24 | 0 | intraperitoneal | 31,1 ± 0,30 | 30,7 ± 0,36 | 30,6 ± 0,37 | 30,3 ± 0,39 |
| 6 | 4 | intraperitoneal | 32,7 ± 0,56 a) | 33,7 ± 0,17 c) | 33,1 ± 0,39 b) | 32,2 ± 0,46 a) |
| 6 | 8 | intraperitoneal | 33,1 ± 0,32 b) | 34,1 ± 0,24 c) | 34,0 ± 0,38 c) | 33,6 ± 0,49 c) |
| 6 | 16 | intraperitoneal | 35,3 ± 0,51 c) | 35,7 ± 0,34 c) | 35,3 ± 0,37 c) | 35,0 ± 0,44 c) |
| 6 | 32 | intraperitoneal | 34,8 ± 0,40 c) | 36,2 ± 0,38 c) | 35,6 ± 0,30 c) | 35,7 ± 0,37 c) |
| 6 | 4 | oral | 30,6 ± 0,30 | 30,6 ± 0,15 | 30,7 ± 0,59 | 30,4 ± 0,67 |
| 12 | 8 | oral | 31,1 ± 0,36 | 31,4 ± 0,56 | 31,5 ± 0,57 | 31,3 ± 0,56 |
| 6 | 16 | oral | 31,4 ± 0,41 | 32,6 ± 0,56 a) | 32,8 ± 0,53 b) | 32,8 ± 0,45 b) |
| 12 | 32 | oral | 31,4 ± 0,31 | 32,6 ± 0,44 c) | 32,9 ± 0,41 c) | 32,8 ± 0,38 c) |
| 6 | 64 | oral | 33,8 ± 0,50 c) | 34,5 ± 0,33 c) | 34,4 ± 0,39 c) | 34,4 ± 0,35 c) |
| 6 | 128 | oral | 33,1 ± 0,56 b) | 34,1 ± 0,56 c) | 34,3 ± 0,58 c) | 34,2 ± 0,53 c) |
| 6 | 256 | oral | 33,5 ± 0,47 c) | 35,2 ± 0,15 c) | 35,3 ± 0,34 c) | 35,1 ± 0,33 c) |

a) = Differenz signifikant auf der Stufe 0,05.
b) = Differenz signifikant auf der Stufe 0,01.
c) = Differenz signifikant auf der Stufe 0,001.

Tabelle IIb

Antireserpinwirkung des gemäß Beispiel IX erhaltenen Produkts IV

| Anzahl Mäuse | mg/kg | Verabreichungsweg | Rektaltemperaturen °C ± SA nach : (Minuten) | | | |
|---|---|---|---|---|---|---|
| | | | 30 | 60 | 90 | 120 |
| 6 | 0 | oral | 30,6 ± 0,39 | 30,6 ± 0,45 | 30,7 ± 0,59 | 30,4 ± 0,67 |
| 6 | 8 | oral | 30,9 ± 0,36 | 32,0 ± 0,56 | 32,2 ± 0,57 | 33,2 ± 0,56 |
| 6 | 16 | oral | 31,1 ± 0,39 | 32,5 ± 0,60[a)] | 32,7 ± 0,51[b)] | 33,1 ± 0,43[b)] |
| 6 | 32 | oral | 31,3 ± 0,31 | 33,0 ± 0,44[c)] | 33,7 ± 0,41[c)] | 33,2 ± 0,38[c)] |
| 6 | 64 | oral | 32,8 ± 0,40[c)] | 34,3 ± 0,32[c)] | 34,5 ± 0,38[c)] | 34,4 ± 0,41[c)] |

a) = Differenz signifikant auf der Stufe 0,05.
b) = Differenz signifikant auf der Stufe 0,01.
c) = Differenz signifikant auf der Stufe 0,001.

Aus den in Tabellen IIa und IIb zusammengefaßten Ergebnissen ist es ersichtlich, daß die durch die erfindungsgemäßen Medikamente ausgeübte antagonistische Wirkung bei dem angewandten Versuchsmodell sehr deutlich ist.

Antiapomorphinwirkung an der Maus:

Zur Zeit 0 erhalten die Mäuse auf oralem Wege das gemäß Beispiel I erhaltene Produkt; 30 Minuten danach verabreicht man ihnen auf subkutanem Wege Apomorphin in einer Dosis von 1 mg · $kg^{-1}$ bzw. 16 mg · $kg^{-1}$. Dann bringt man die Mäuse in einzelne Kästen (11 × 3,5 × 4 cm), die deren Beobachtung gestatten. Die Rektaltemperatur wird 30 Minuten nach der Verabreichung des Apomorphins gemessen. Kontrolltiere erhalten destilliertes Wasser anstelle des gemäß Beispiel II erhaltenen erfindungsgemäßen Produkts. Die bei den Kontrolltieren beobachtete durchschnittliche Hypothermie betrug 34,9° C bei den Tieren, die eine Apomorphindosis von 1 mg · $kg^{-1}$ erhalten hatten, und 33,5° C bei denen mit 16 mg · $kg^{-1}$. Die prozentuale Hemmung der Hypothermie wird für jede Dosis des erfindungsgemäßen Medikaments berechnet. Die Ergebnisse sind in der nachfolgenden Tabelle III ausgedrückt. Sie zeigen, daß das erfindungsgemäße Medikament einen stärkeren Antagonismus gegenüber den Wirkungen einer hohen Dosis Apomorphin aufweist als gegenüber denen einer schwachen Dosis desselben.

Bei genügenden Dosierungen des erfindungsgemäßen Medikaments ist der Antagonismus gegen durch Apomorphin induzierte Hypothermie praktisch vollständig. Dagegen sind die stereotypen Bewegungen und das Aufstehen, die durch Apomorphin hervorgerufen werden, unverändert bei den Tieren, die das erfindungsgemäße Medikament erhielten, ganz gleich in welcher Dosis.

Tabelle III

Einfluß der erfindungsgemäßen Medikamente auf die durch Apomorphin hervorgerufene Hypothermie

| Anzahl Mäuse | mg/kg | Verabreichungsweg | Rektaltemperaturen Ergebnisse als Prozenthemmung der Hypothermie<br>Apomorphin 1 mg · $kg^{-1}$ subkutan | <br>Apomorphin 16 mg · $kg^{-1}$ subkutan |
|---|---|---|---|---|
| 6 | 0,5 | intraperitoneal | 7 | −5 |
| 12 | 2 | intraperitoneal | 3 | 45 |
| 6 | 4 | intraperitoneal | −13 | 39 |
| 6 | 16 | intraperitoneal | 10 | 51 |
| 12 | 4 | oral | 0 | 31 |
| 12 | 8 | oral | −2 | 40 |
| 12 | 16 | oral | 0,5 | 42 |
| 12 | 32 | oral | 45 | 77 |
| 6 | 64 | oral | 51 | 86 |
| 6 | 128 | oral | 68 | 81 |
| 6 | 256 | oral | 89 | 116 |

*Antioxotremorinwirkung an der Maus:*

Zur Zeit 0 erhalten die Mäuse auf intraperitonealem Wege Oxotremorin (0,5 mg · $kg^{-1}$); 30 Minuten danach verabreicht man ihnen auf intraperitonealem oder oralem Wege entweder destilliertes Wasser oder das nach Beispiel I erhaltene erfindungsgemäße Medikament.

Dann beobachtet man die Mäuse und bewertet das Zittern 30 Minuten lang alle 10 Minuten von 0 bis 3 je nach dessen Intensität. Ferner beobachtet man 2 Stunden lang, ob, mit oder ohne Zittern, periphere Symptome vorliegen (Tränen, übermäßiger Speichelfluß, Darmentleerungen). Die Rektaltemperatur wird nach der Verabreichung des Oxotremorins 2 Stunden lang alle 30 Minuten gemessen.

Die Ergebnisse bezüglich der Temperatur sind in der nachfolgenden Tabelle IV angeführt. Sie zeigen deutlichen Antagonismus. Dagegen hat das nach Beispiel II erhaltene erfindungsgemäße Medikament bei den untersuchten Dosierungen auf oralem oder intraperitonealem Wege gar keine Änderung des durch Oxotremorin hervorgerufenen Zitterns bzw. der peripheren Symptome zur Folge.

Tabelle IV

Einfluß des erfindungsgemäßen Medikaments auf die durch Oxotremorin hervorgerufene Hypothermie bei der Maus

| Anzahl Mäuse | mg/kg | Verabreichungsweg | Rektaltemperaturen °C nach: (Minuten) | | | |
|---|---|---|---|---|---|---|
| | | | 30 | 60 | 90 | 120 |
| 36 | 0 | | 30,1 ± 0,20 | 20,7 ± 0,24 | 28,4 ± 0,30 | 29,5 ± 0,55 |
| 6 | 2 | ip | 30,8 ± 0,43 | 29,1 ± 0,46 | 29,4 ± 0,52 | 29,9 ± 0,77 |
| 6 | 4 | ip | 30,2 ± 0,36 c) | 31,8 ± 0,42 c) | 33,5 ± 0,49 c) | 35,7 ± 0,40 c) |
| 6 | 8 | ip | 32,1 ± 0,52 c) | 31,3 ± 0,52 c) | 32,2 ± 0,61 c) | 33,8 ± 0,91 c) |
| 12 | 16 | ip | 32,9 ± 0,46 c) | 32,0 ± 0,65 c) | 32,8 ± 0,60 c) | 35,9 ± 0,81 c) |
| 6 | 32 | ip | 32,0 ± 0,70 c) | 31,2 ± 0,73 c) | 32,2 ± 0,05 c) | 33,2 ± 1,2 b) |
| 6 | 64 | ip | 34,0 ± 0,87 c) | 33,0 ± 1,2 c) | 33,7 ± 1,15 c) | 34,5 ± 1,0 c) |
| 6 | 1 | oral | 29,8 ± 0,40 | 27,0 ± 0,65 | 27,8 ± 1,36 | 28,0 ± 1,65 |
| 6 | 2 | oral | 31,5 ± 0,43 b) | 31,0 ± 0,71 c) | 31,2 ± 0,86 c) | 31,9 ± 1,14 |
| 12 | 4 | oral | 31,0 ± 0,36 c) | 31,0 ± 0,34 c) | 31,6 ± 0,91 c) | 32,4 ± 0,89 b) |
| 12 | 0 | oral | 32,6 ± 0,32 c) | 32,4 ± 0,29 c) | 32,9 ± 0,38 c) | 33,0 ± 0,56 c) |
| 12 | 16 | oral | 31,9 ± 0,53 c) | 31,9 ± 0,49 c) | 32,8 ± 0,70 c) | 33,6 ± 0,97 c) |
| 12 | 32 | oral | 33,0 ± 0,60 c) | 32,7 ± 0,63 c) | 32,8 ± 0,62 c) | 33,7 ± 0,95 c) |
| 12 | 64 | oral | 32,2 ± 0,32 c) | 31,7 ± 0,52 c) | 32,8 ± 0,73 c) | 34,1 ± 0,62 c) |
| 6 | 129 | oral | 34,5 ± 0,80 c) | 34,9 ± 0,59 c) | 36,2 ± 0,82 c) | 37,1 ± 0,77 c) |
| 6 | 256 | oral | 32,4 ± 0,63 c) | 31,3 ± 0,73 c) | 32,4 ± 1,07 c) | 33,9 ± 1,30 b) |

ip = intraperitoneal.
b) = Differenz signifikant auf der Stufe 0,01.
c) = Differenz signifikant auf der Stufe 0,001.

### Wirkung auf die Toxizität von Yohimbin an der Maus:

Zur Zeit 0 erhalten die Mäuse auf oralem Wege entweder destilliertes Wasser oder das erfindungsgemäße Medikament; 30 Minuten danach verabreicht man ihnen auf subkutanem Wege Yohimbinchlorhydrat (25 mg · $kg^{-1}$). Die Sterbeziffer wird 4 Stunden lang alle Stunde bestimmt und nach 24 Stunden aufgezeichnet.

Die in den nachfolgenden Tabellen Va und Vb angeführten Ergebnisse zeigen eine Potenzierung der Toxizität des Yohimbins durch die erfindungsgemäßen Medikamente, wobei die Potenzierung ab einer Dosis von 16 mg · $kg^{-1}$ erheblich ist. Zwischen den nach 4 und nach 24 Stunden gefundenen Zahlen besteht kein Unterschied.

Tabelle Va

Einfluß des nach Beispiel II erhaltenen erfindungsgemäßen Medikaments auf die Toxizität von Yohimbin an der Maus

| Anzahl Mäuse | mg/kg | Sterbeziffer in % 4 und 24 Stunden nach Verabreichung von Yohimbin | |
|---|---|---|---|
| 30 | 0 | 0 | 0 |
| 20 | 2 | 0 | 0 |
| 10 | 4 | 0 | 0 |
| 20 | 8 | 10 | 10 |
| 20 | 16 | 20 | 20 |
| 20 | 32 | 25 | 25 |
| 20 | 64 | 30 | 30 |
| 10 | 128 | 60 | 60 |
| 10 | 256 | 70 | 70 |

Tabelle Vb

Einfluß des nach Beispiel IX (Produkt IV) erhaltenen erfindungsgemäßen Medikaments auf die Toxizität von Yohimbin an der Maus (10 Mäuse pro Dosis)

| mg/kg oral verabreicht | Sterbeziffer in % 4 und 24 Stunden nach Verabreichung von Yohimbin | |
|---|---|---|
| | 4 | 24 |
| 0 | 0 | 0 |
| 2 | 10 | 10 |
| 4 | 20 | 20 |
| 8 | 30 | 30 |
| 16 | 70 | 70 |
| 32 | 80 | 80 |
| 64 | 100 | 100 |
| 128 | 80 | 80 |
| 256 | 100 | 100 |

Wechselwirkung mit Barbitalnatrium:

Zur Zeit 0 erhalten die Mäuse auf intraperitonealem oder oralem Wege destilliertes Wasser oder das erfindungsgemäße Medikament. 30 Minuten danach verabreicht man ihnen auf intraperitonealem Wege Barbitalnatrium (180 mg · $kg^{-1}$). Die Zeiten bis zur Unterdrückung und zum Wiedererscheinen des Aufstehreflexes werden für jedes Tier aufgezeichnet.

Die erhaltenen Ergebnisse sind in der nachfolgenden Tabelle VI angeführt:

Tabelle VI

Einfluß des nach Beispiel I erhaltenen erfindungsgemäßen Medikaments auf den durch Barbital-Natrium (180 mg · $kg^{-1}$) hervorgerufenen Schlaf induzierten an der Maus

| Anzahl Mäuse | mg/kg | Verabreichungsweg | % Mäuse, die geschlafen haben | Zeit in Minuten | |
|---|---|---|---|---|---|
| | | | | Einschlafen | Schlaf |
| 6 | 0 | ip | 100 | 21 | 88 ± 8 |
| 6 | 2 | ip | 100 | 27 | 67 ± 16 |
| 6 | 4 | ip | 83 | 22 | 37 ± 13 |
| 6 | 8 | ip | 83 | 30 | 25 ± 4 |
| 6 | 16 | ip | 33 | 38 | 14 |
| 6 | 32 | ip | 0 | 0 | 0 |
| 12 | 0 | oral | 100 | 29 | 64 ± 9 |
| 6 | 2 | oral | 83 | 25 | 47 ± 15 |
| 12 | 4 | oral | 25 | 33 | 56 |
| 12 | 8 | oral | 42 | 32 | 43 |
| 6 | 16 | oral | 0 | 0 | 0 |
| 6 | 32 | oral | 00 | 0 | 0 |

ip = intraperitoneal.

Das erfindungsgemäße Medikament wirkt bei oraler oder intraperitonealer Verabreichung dem durch Barbital hervorgerufenen Schlaf deutlich entgegen.

»Antiermüdungswirkung« an der Ratte:

Die von Boissier und Simon (Thérapie 1968, 23, 1267—1276) beschriebene Methode wird auf zuvor ermüdete Ratten angewandt, die sich in einem Pendelkasten mit zwei Abteilen befinden. Das erfindungsgemäße Medikament wird 6 Ratten auf oralem Wege und 6 weiteren auf intraperitonealem Wege verabreicht. Unter diesen Bedingungen beobachtet man bei allen Tieren eine Wiederaufnahme der konditionierten Aktivität, wobei die Wiederaufnahme statistisch signifikant ist und im Durchschnitt 2 Stunden lang anhält.

Weitere durchgeführte Tests bezüglich gewisser Wirkungen der erfindungsgemäßen Medikamente verliefen negativ. Diese sind insofern von Bedeutung, als sie es gestatten, die Wirkungsbreite dieser Substanzen abzugrenzen und mit der anderer, bekannter psychotroper Antidepressivsubstanzen zu vergleichen. Außerdem sind sie von Bedeutung insofern, als daß sie es ermöglichen, das Fehlen einer Veränderung in einer bestimmten Anzahl normaler Verhaltungsweisen aufzuzeigen.

a) Fehlen einer Störung der Ausweichkonditionierung:

Man verwendet einen einfachen Test auf konditionierte Hemmung, nämlich den Vierplattentest (Aron und Mitarbeiter, Neuropharmacology, 1971, 10, 459—470). Mit einer Dosis von 8 bzw. 32 mg · $kg^{-1}$ (10 Tiere pro Gruppe) beobachtet man keine Veränderung der konditionierten Hemmung an der Maus. Dagegen beobachtet man bei diesem Test eine leicht erhöhte Leistung der Tiere, aber diese scheint der oben angegebenen stimulierenden Wirkung zuzuschreiben zu sein.

b) Fehlen einer Veränderung in den Wirkungen des Pentobarbitals:

Zur Zeit 0 erhalten die Mäuse auf intraperitonealem oder oralem Wege destilliertes Wasser oder das gemäß Beispiel I erhaltene erfindungsgemäße Medikament. 30 Minuten danach verabreicht man ihnen auf intraperitonealem Wege Pentobarbital (50 mg · $kg^{-1}$). Die Zeit bis zur Unterdrückung und zum Wiederauftreten des Aufstehreflexes werden für jedes Tier aufgezeichnet.

Die Ergebnisse sind in der nachfolgenden Tabelle VII angeführt:

Tabelle VII

Einfluß des erfindungsgemäßen, gemäß Beispiel I erhaltenen Medikaments auf durch Pentobarbital (50 mg · $kg^{-1}$ Intraperitoneal) hervorgerufenen Schlaf an der Maus

| Anzahl Mäuse | mg/kg | Verabreichungsweg | % Mäuse, die geschlafen haben | Zeit in Minuten | |
|---|---|---|---|---|---|
| | | | | Einschlafen | Schlaf |
| 30 | 0 | ip | 100 | 4 | 48 ± 4 |
| 6 | 2 | ip | 10 | 4 | 47 ± 7 |
| 6 | 4 | ip | 100 | 4 | 55 ± 8 |
| 12 | 8 | ip | 100 | 4 | 36 ± 3 |
| 6 | 16 | ip | 83 | 4,5 | 26 ± 7 |
| 12 | 32 | ip | 92 | 4,5 | 27 ± 3 |
| 6 | 64 | ip | 100 | 4 | 30 ± 9 |
| 6 | 128 | ip | 83 | 6 | 25 ± 5 |
| 30 | 0 | oral | 100 | 4 | 48 ± 4 |
| 6 | 2 | oral | 100 | 4 | 60 ± 8 |
| 6 | 4 | oral | 100 | 4 | 45 ± 4 |
| 6 | 8 | oral | 100 | 4 | 57 ± 10 |
| 6 | 16 | oral | 100 | 4 | 41 ± 7 |
| 6 | 32 | oral | 100 | 4 | 49 ± 4 |
| 6 | 64 | oral | 100 | 5 | 29 ± 8 |
| 6 | 128 | oral | 100 | 4 | 38 ± 7 |

ip = intraperitoneal.

Bis zu einer Dosis von 128 mg · $kg^{-1}$ auf intraperitonealem oder oralem Wege führt das erfindungsgemäße Medikament zu keinem deutlichen Antagonismus gegenüber der schlaffördernden Wirkung des Pentobarbitals.

c) Fehlen einer Hemmwirkung auf Monoaminoxydase:

Bei einer Dosis von 16 bzw. 64 mg · $kg^{-1}$ auf oralem Wege bestätigt es sich, daß das erfindungsgemäße Medikament keine Potenzierung der Wirkungen einer geringeren als der krampferregenden Dosis Tryptamin (3 mg · $kg^{-1}$ intravenös) an der Ratte zur Folge hat.

Untersuchung antagonistischer Wirkungen der an der Ratte durch das erfindungsgemäße Medikament hervorgerufenen Stereotypien

a) Einfluß von Pimozid

Zur Zeit 0 verabreicht man auf intraperitonealem Wege Pimozid (1 mg · $kg^{-1}$) oder destilliertes Wasser; 30 Minuten danach erhalten sämtliche Tiere auf intraperitonealem Wege das gemäß Beispiel III erhaltene erfindungsgemäße Medikament (8 mg · $kg^{-1}$). Die Stereotypien werden wie oben beschrieben ausgewertet. Man berechnet die Fläche unter den Stereotypiekurven und erhält dabei:

| | |
|---|---|
| Fläche unter der Kurve für das Medikament allein | 63 |
| Fläche unter der Kurve für das nach Pimozid verabreichte Medikament: | 17. |

Unter den genannten Versuchsbedingungen antagonisierte Pimozid die durch das erfindungsgemäße Medikament hervorgerufenen Stereotypien in sehr erheblichem Ausmaß.

b) Einfluß von $\alpha$-Methyltyrosin

Zur Zeit 0 erhalten die Tiere auf intraperitonealem Wege entweder destilliertes Wasser oder $\alpha$-Methyltyrosin in einer Dosis von 64 mg · $kg^{-1}$; 150 Minuten danach verabreicht man ihnen auf intraperitonealem Wege das gemäß Beispiel III erhaltene erfindungsgemäße Medikament in einer Dosis von 8 mg · $kg^{-1}$.

Man beobachtet die Tiere unter den weiter oben bei der Untersuchung der Stereotypien an der Ratte beschriebenen Bedingungen.

Unter diesen Versuchsbedingungen hat eine Vorbehandlung mit $\alpha$-Methyltyrosin praktisch keinen Einfluß auf die durch das erfindungsgemäße Medikament hervorgerufenen Stereotypien (Fläche unter der Kurve für das Medikament allein: 63; Medikament + $\alpha$-Methyltyrosin: 60,8).

Vergleich der Wirksamkeiten der Isomeren

Einige Versuche werden durchgeführt, um die Wirkungen des trans-Isomeren folgender Zusammensetzung zu vergleichen:

a) 100% Diastereoisomer $\alpha$
b) 100% Diastereoisomer $\beta$
c) Gemisch (80/20) der beiden Diastereoisomeren.

Die akuten Toxizitäten der 3 Verbindungen sind ähnlich, wobei die $LD_{50}$ an der Maus bei oraler Verabreichung zwischen 800 und 1200 mg/kg liegt.

Der Vergleich der Aktivitäten erfolgt mit äquivalenten Dosierungen (32 mg/kg), die der Ratte bzw. der Maus auf oralem Wege verabreicht werden. Dabei handelt es sich um die Messung der akuten Toxizitäten und um die Prüfungen, die es gestatten, die psychoanaleptischen Wirkungen (Motilität an der Maus, stereotype Bewegungen an der Ratte) zu ermitteln, sowie um die Tests, die es ermöglichen, die antidepressiven Wirkungen (Wechselwirkungen mit Reserpin und Apomorphin) darzulegen.

Die Ergebnisse der Prüfungen zeigen keine hohe Korrelation und können wie in der nachfolgenden Tabelle VII schematisch dargestellt werden:

Tabelle VII

| Isomer, 32 mg/kg (orale Verabreichung) | Erhöhung der Motilität der Maus | Stereotypie erzeugende Wirkung an der Ratte | Antireserpinwirkung | Antiapomorphinwirkung |
|---|---|---|---|---|
| 100% $\alpha$ | + | 0 | ± | ++ |
| 100% $\beta$ | +++ | +++ | +++ | +++ |
| 80% $\alpha$ und 20% $\beta$ | ++ | ++ | +++ | +++ |

Das Diastereoisomer $\alpha$ scheint sich also erheblich von $\beta$ zu unterscheiden: die Antiapomorphinwirkung bleibt bestehen, während die Antireserpinwirkung und die Erhöhung der Motilität der Maus weniger ausgeprägt sind und die Stereotypie erzeugende Wirkung an der Ratte praktisch verschwindet.

Verglichen mit dem Gemisch aus 80% $\alpha$ und 20% $\beta$ scheint das Diastereoisomer $\beta$ eine größere Wirksamkeit und insbesondere eine stärkere stimulierende Wirkung aufzuweisen.

Schlußfolgerung:

Die Wirkungen der erfindungsgemäßen Medikamente lassen sich wie folgt schematisch einordnen:

1. Erhebliche stimulierende Wirkung,
   gekennzeichnet durch eine Erhöhung der Motilität, die Gegenwart von Stereotypien, eine Antiermüdungswirkung und eine Antischlafwirkung gegenüber Barbital, jedoch nicht gegenüber Pentobarbital. Diese stimulierende Wirkung wird durch Pimozid, einen Blocker der dopaminergischen Rezeptoren, antagonisiert, jedoch durch $\alpha$-Methyltyrosin, einen Inhibitor der Catecholaminsynthese, nicht verändert. Sie scheint sich also in zwei Merkmalen von derjenigen der Substanzen der Amphetamingruppe zu unterscheiden: Abwesenheit von Antagonismus gegenüber der schlaffördernden Wirkung des Pentobarbitals und Fehlen der Unterdrückung unter dem Einfluß von $\alpha$-Methyltyrosin.

2. Antidepressivartige Wirkung

   In den Tests besitzen die erfindungsgemäßen Medikamente Wirkungen, die als bezeichnend für eine antidepressive Wirksamkeit angesehen werden: Antireserpinwirkung, antagonistische Wirkung auf die durch Apomorphin hervorgerufene Hypothermie (und wie die Imipramine weist das erfindungsgemäße Medikament eine stärkere Gegenwirkung auf die Effekte einer hohen Dosis Apomorphin als auf die einer kleinen Dosis auf), antagonistische Wirkung gegenüber der durch Oxotremorin hervorgerufenen Hypothermie und Potenzierung der Toxizität des Yohimbins an der Maus.

3. Fehlen einer Hemmwirkung auf Monoaminoxydase (Tryptamintest).

4. Fehlen von peripheren anticholinergischen Wirkungen:
   keine Veränderung in den an der Maus durch Oxotremorin hervorgerufenen peripheren Symptomen.

Gemäß den Versuchsergebnissen lassen sich die erfindungsgemäßen Medikamente eindeutig unter die psychotropen Substanzen mit antidepressiver Wirkung einreihen. Sie besitzen zudem eine deutliche psychostimulierende Wirkung, welche sich in ihrem Mechanismus von derjenigen der Nooanaleptika oder der Amphetamine unterscheidet. Durch die Abwesenheit einer Hemmwirkung auf MAO und einer peripheren anticholinergischen Wirkung sind sie deutlich von den beiden großen, bekannten Gruppen von Antidepressiva, nämlich den Imipraminen und IMAO, differenziert.

Die erfindungsgemäßen Medikamente der allgemeinen Formel IIIa oder IIIb lassen sich auf beliebigen Verabreichungswegen in Dosierungen zwischen 10 und 500 mg/Tag beim Erwachsenen anwenden.

Aus der vorangehenden Beschreibung ist ersichtlich, daß man, unabhängig von der gewählten Art und Weise der Ausführung, Verwirklichung und Anwendung, Medikamente mit einer antidepressiven und psychostimulierenden Wirkung erhält, die eine weite therapeutische Anwendung gestatten sollte, insbesondere:

— in allen Fällen: Wirkung auf sämtliche depressiven Zustände, gleich ob diese endogenen Ursprungs oder Reaktionsursprungs sind und gleich bei welchem Alter;

— bei Kindern: Schwierigkeiten mit intellektueller Fixation, Apathie, Asthenie, Überarbeitung, Gedächtnisstörungen und Schwierigkeiten beim Fixieren der Aufmerksamkeit;

— bei Erwachsenen und alten Leuten: Asthenie, Herabsetzung der intellektuellen Aktivität, Apathie, Verbesserung von Sedationszuständen, die durch die Verabreichung von antiepileptischen Medikamenten, Beruhigungsmitteln, Neuroleptika oder anderen hervorgerufen wurden, und Apathie bei Parkinsonscher Krankheit.

Wie sich aus dem Vorangehenden ergibt, ist die Erfindung keineswegs auf jene Arten der Ausführung, Verwirklichung und Anwendung beschränkt, die eben mehr im einzelnen beschrieben

wurden; im Gegenteil umfaßt sie sämtliche Varianten, die sich einem Fachmann bieten können, ohne vom Ziel und Rahmen der vorliegenden Erfindung abzugehen.

## Patentansprüche für die Vertragsstaaten BE, CH, DE, FR, GB, IT, NL, SE

1. Neue halogenierte δ-Hydrocycarbonsäurederivate, dadurch gekennzeichnet, daß sie der untenstehenden allgemeinen Formel IIIa

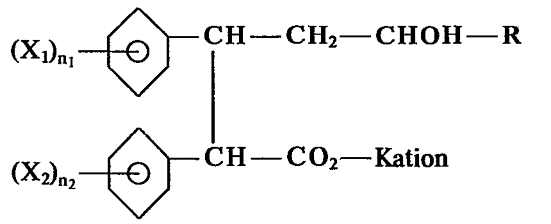

(IIIa)

(offenen Form in basischem Medium)

oder der untenstehenden allgemeinen Formel IIIb:

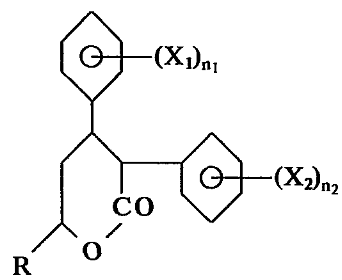

(IIIb)

(zyklisierte Form in saurem Medium)

entsprechen, worin $X_1$ und $X_2$ Halogenatome darstellen, $n_1$ und $n_2$ ganze Zahlen zwischen 0 und 5 bedeuten, wobei jedoch im Fall von $n_1 = 0$ $n_2$ von 0 verschieden ist, bzw. umgekehrt, R ein Wasserstoffatom oder eine Alkyl- oder Arylgruppe ist und Kation ein pharmazeutisch verträgliches, sich von einer Base herleitendes Ion oder Metallion darstellt.

2. Neues halogeniertes δ-Hydroxycarbonsäurederivat nach Anspruch 1, dadurch gekennzeichnet, daß es aus 4-(4'-Chlorphenyl)-6-methyl-3-phenyl-3,4,5,6-tetrahydropyron-2 besteht.

3. Neues halogeniertes δ-Hydroxycarbonsäurederivat nach Anspruch 1, dadurch gekennzeichnet, daß es aus dem Natriumsalz der 3-(4'-Chlorphenyl)-5-hydroxy-2-phenylhexansäure besteht.

4. Derivate nach einem der Ansprüche 1 bis 3, dadurch gekennzeichnet, daß sie stereochemisch rein sind.

5. Medikamente mit antidepressiver psychostimulierender Wirkung, dadurch gekennzeichnet, daß sie als Wirkstoffe halogenierte δ-Hydroxycarbonsäurederivate nach einem der Ansprüche 1 bis 4 enthalten.

6. Medikamente nach Anspruch 5, dadurch gekennzeichnet, daß sie stereochemisch rein sind.

7. Verfahren zur Herstellung von Verbindungen nach Anspruch 1, dadurch gekennzeichnet, daß man im Verlauf einer ersten Stufe ein halogeniertes Ketonitril herstellt, welches dann durch Hydrolyse und Reduktion in die entsprechende Verbindung der allgemeinen Formel III, in basischem Medium in der offenen Form (a) oder in saurem Medium in der zyklisierten Form (b), umgewandelt wird.

8. Verfahren nach Anspruch 7, dadurch gekennzeichnet, daß man das halogenierte Ketonitril zunächst zum entsprechenden halogenierten Hydroxynitril reduziert und dieses dann in Gegenwart einer Säure zyklisiert und hydrolysiert, um die Verbindung der allgemeinen Formel IIIb zu erhalten.

9. Verfahren nach Anspruch 7, dadurch gekennzeichnet, daß man anfänglich das Ketonitril durch Hydrolyse in Gegenwart von Säure in die entsprechende halogenierte Ketosäure umwandelt und anschließend die Reduktion und Zyklisierung durchführt, um eine Verbindung der allgemeinen Formel IIIb zu erhalten.

10. Verfahren nach Anspruch 7, dadurch gekennzeichnet, daß man das halogenierte Ketonitril zunächst durch saure Hydrolyse in eine Ketosäure umwandelt, welche dann mit einer pharmazeutisch verträglichen Base in ein Ketosalz überführt wird, und anschließend die Reduktion vornimmt, um eine Verbindung der allgemeinen Formel IIIa zu erhalten.

11. Verfahren nach Anspruch 7, dadurch gekennzeichnet, daß man das halogenierte Ketonitril durch

Säurebehandlung in Gegenwart eines Alkohols in einen Ketoester umwandelt, diesen dann zum entsprechenden Hydroester reduziert und schließlich mit einer pharmazeutisch verträglichen Base hydrolysiert, um eine Verbindung der allgemeinen Formel IIIa zu erhalten.

12. Verfahren nach anspruch 11, dadurch gekennzeichnet, daß man zur Bildung eines Ketosalzes den Ketoester zunächst mit einer pharmazeutisch verträglichen Base behandelt und anschließend die Reduktion vornimmt, um eine Verbindung der allgemeinen Formel IIIa zu erhalten.

13. Verfahren nach Anspruch 7, dadurch gekennzeichnet, daß man das halogenierte Ketonitril durch alkalische Hydrolyse in das entsprechende Ketosalz überführt und dann die Reduktion vornimmt.

14. Verfahren nach einem der Ansprüche 7 bis 13, dadurch gekennzeichnet, daß man das halogenierte Ketonitril durch Einwirkung eines $\alpha$-äthylenischen Ketons, das der untenstehenden allgemeinen Formel IV

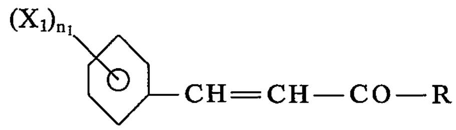

(IV)

entspricht, auf Benzylcyanid der untenstehenden allgemeinen Formel V:

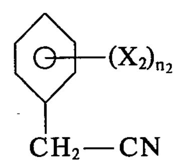

(V)

um eine Verbindung der untenstehenden allgemeinen Formel VI:

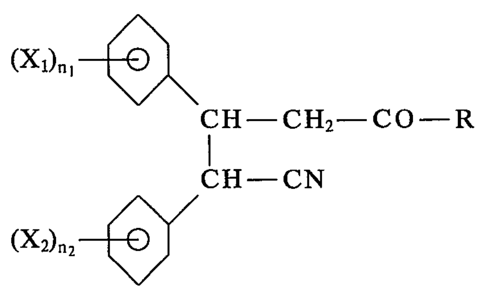

(VI)

worin $X_1$, $X_2$, $n_1$, $n_2$ und R dieselbe Bedeutung wie oben haben, zu erhalten, herstellt.

15. Verfahren zur Herstellung von halogenierten $\delta$-Hydroxycarbonsäurederivaten, nach einem der Ansprüche 7 bis 14, dadurch gekennzeichnet, daß man im Verlauf jeder Synthesestufe jeweils mehrere Umkristallisationen aus geeigneten Lösungsmitteln vornimmt, um die trans-(threo)-Derivate von den cis-(erythro)-Derivaten zu trennen oder eines dieser trans- bzw. cis-Derivate im Gemisch anzureichern.

16. Verfahren anch Anspruch 15, dadurch gekennzeichnet, daß man die aus Hydroxynitrilen, Ketonitrilen, Ketosäuren bzw. Ketoestern bestehenden Zwischenprodukte sowie die Pyrone aus polaren Lösungsmitteln und die aus Hydroxyestern bestehenden Zwischenprodukte aus sauerstofffreien polaren Lösungsmitteln umkristallisiert, wogegen die Ketosalze und Hydrosalze aus mindestens 10% Wasser enthaltenden polaren Lösungsmitteln umkristallisiert werden.

17. Verfahren nach einem der Ansprüche 7 bis 14, dadurch gekennzeichnet, daß die Zyklisierung in saurem Medium zur Bildung der Derivate der allgemeinen Formel III bei Raumtemperatur stattfindet, wenn das gesuchte Diastereoisomer die $\beta$-Form ist, bzw. bei Rückflußtemperatur vorgenommen wird, wenn die Bildung des Diastereoisomeren der $\alpha$-Form erwünscht ist.

18. Verfahren nach einem der Ansprüche 7 bis 17, dadurch gekennzeichnet, daß die Reduktion der Ketoester bzw. Ketosalze zu den entsprechenden Hydroxyestern bzw. Hydroxysalzen mit Hilfe von $NaBH_4$ in wäßriger Lösung erfolgt.

**Patentansprüche für den Vertragsstaat AT**

1. Verfahren zur Herstellung von neuen halogenierten $\delta$-Hydroxycarbonsäurederivaten der Formel IIIa

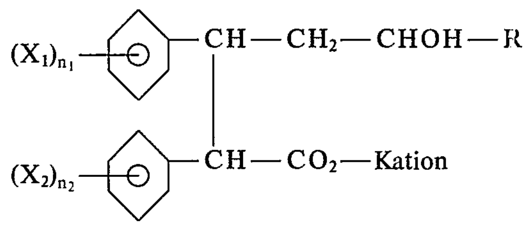

(IIIa)

(offenen Form in basischem Medium)

oder der Formel IIIb:

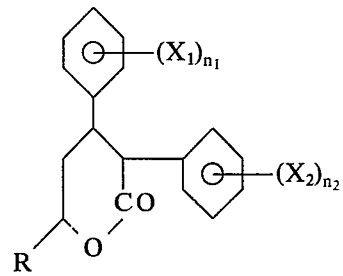

(IIIb)

(zyklisierte Form in saurem Medium)

worin $X_1$ und $X_2$ Halogenatome darstellen, $n_1$ und $n_2$ ganze Zahlen zwischen 0 und 5 bedeuten, wobei jedoch im Fall von $n_1 = 0$ $n_2$ von 0 verschieden ist, bzw. umgekehrt., R ein Wasserstoffatom oder eine Alkyl- oder Arylgruppe ist und Kation ein pharmazeutisch verträgliches, sich von einer Base herleitendes Ion oder Metallion darstellt, dadurch gekennzeichnet, daß man ein halogeniertes Ketonitril durch Hydrolyse und Reduktion in die entsprechende Verbindung der Formel III, in basischem Medium in der offenen Form (a) oder in saurem Medium in der zyklisierten Form (b), umwandelt.

2. Verfahren nach Anspruch 1, dadurch gekennzeichnet, daß man das halogenierte Ketonitril zunächst zum entsprechenden halogenierten Hydroxynitril reduziert und dieses dann in Gegenwart einer Säure zyklisiert und hydrolysiert, um die Verbindung der allgemeinen Formel IIIb zu erhalten.

3. Verfahren nach Anspruch 1, dadurch gekennzeichnet, daß man anfänglich das Ketonitril durch Hydrolyse in Gegenwart von Säure in die entsprechende halogenierte Ketosäure umwandelt und anschließend die Reduktion und Zyklisierung durchführt, um eine Verbindung der allgemeinen Formel IIIb zu erhalten.

4. Verfahren nach Anspruch 1, dadurch gekennzeichnet, daß man das halogenierte Ketonitril zunächst durch saure Hydrolyse in eine Ketosäure umwandelt, welche dann mit einer pharmazeutisch verträglichen Base in ein Ketosalz überführt wird, und anschließend die Reduktion vornimmt, um eine Verbindung der allgemeinen Formel IIIa zu erhalten.

5. Verfahren nach Anspruch 1, dadurch gekennzeichnet, daß man das halogenierte Ketonitril durch Säurebehandlung in Gegenwart eines Alkohols in einen Ketoester umwandelt, diesen dann zum entsprechenden Hydroxyester reduziert und schließlich mit einer pharmazeutisch verträglichen Base hydrolysiert, um eine Verbindung der allgemeinen Formel IIIa zu erhalten.

6. Verfahren nach Anspruch 5, dadurch gekennzeichnet, daß man zur Bildung eines Ketosalzes den Ketoester zunächst mit einer pharmazeutisch verträglichen Base behandelt und anschließend die Reduktion vornimmt, um eine Verbindung der allgemeinen Formel IIIa zu erhalten.

7. Verfahren nach Anspruch 1, dadurch gekennzeichnet, daß man das halogenierte Ketonitril durch alkalische Hydrolyse in das entsprechende Ketosalz überführt und dann die Reduktion vornimmt.

8. Verfahren nach einem der Ansprüche 1 bis 7, dadurch gekennzeichnet, daß man das halogenierte Ketonitril durch Einwirkung eines $\alpha$-äthylenischen Ketons, das der untenstehenden allgemeinen Formel IV

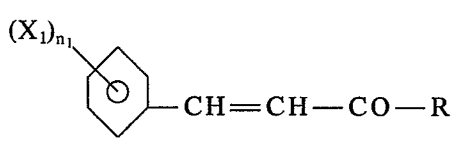

(IV)

entspricht, auf Benzylcyanid der allgemeinen Formel V:

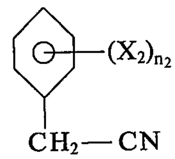

(V)

um eine Verbindung der untenstehenden allgemeinen Formel VI:

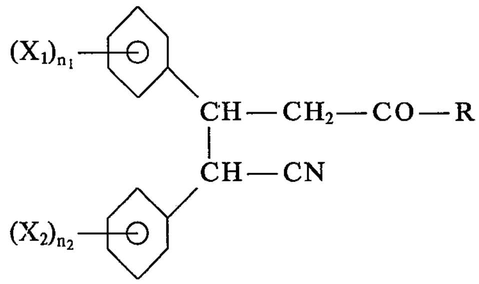

(VI)

worin $X_1$, $X_2$, $n_1$, $n_2$ und R dieselbe Bedeutung wie oben haben, zu erhalten, herstellt.

9. Verfahren zur Herstellung von halogenierten δ-Hydroxycarbonsäurederivaten, nach einem der Ansprüche 1 bis 8, dadurch gekennzeichnet, daß man im Verlauf jeder Synthesestufe jeweils mehrere Umkristallisationen aus geeigneten Lösungsmitteln vornimmt, um die trans-(threo)-Derivate von den cis-(erythro)-Derivaten zu trennen oder eines dieser trans- bzw. cis-Derivate im Gemisch anzureichern.

10. Verfahren nach Anspruch 9, dadurch gekennzeichnet, daß man die aus Hydroxynitrilen, Ketonitrilen, Ketosäuren bzw. Ketoestern bestehenden Zwischenprodukte sowie die Pyrone aus polaren Lösungsmitteln und die aus Hydroxyestern bestehenden Zwischenprodukte aus sauerstofffreien polaren Lösungsmitteln umkristallisiert, wogegen die Ketosalze und Hydroxysalze aus mindestens 10% Wasser enthaltenden polaren Lösungsmitteln umkristallisiert werden.

11. Verfahren nach einem der Ansprüche 1 bis 8, dadurch gekennzeichnet, daß die Zyklisierung in saurem Medium zur Bildung der Derivate der allgemeinen Formel III bei Raumtemperatur stattfindet, wenn das gesuchte Diastereoisomer die β-Form ist, bzw. bei Rückflußtemperatur vorgenommen wird, wenn die Bildung des Diastereoisomeren der α-Form erwünscht ist.

12. Verfahren nach einem der Ansprüche 1 bis 11, dadurch gekennzeichnet, daß die Reduktion der Ketoester bzw. Ketosalze zu den entsprechenden Hydroxyestern bzw. Hydroxysalzen mit Hilfe von $NaBH_4$ in wäßriger Lösung erfolgt.

**Claims for the contracting states BE, CH, DE, FR, GB, IT, NI, SE**

1. New halogen derivatives of δ-hydroxycarboxylic acids, which correspond to the general formula IIIa below:

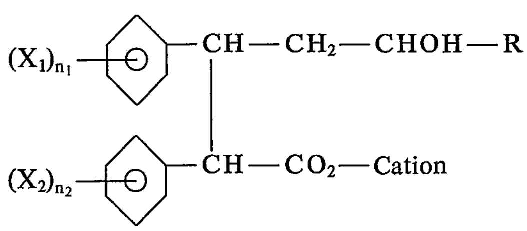

(IIIa)

(open form, in a basic medium)

or to the general formula IIIb below:

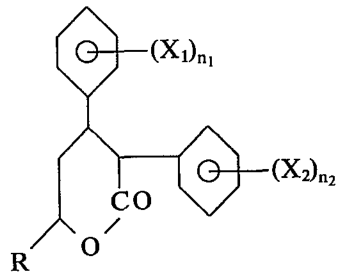

(IIIb)

(cyclized form, in an acid medium)

wherein $X_1$ and $X_2$ are halogen atoms, $n_1$ and $n_2$ are integers between 0 and 5, but, if $n_1 = 0$, $n_2$ is different from 0, and vice versa, R is a hydrogen atom or an alkyl or aryl group, and Cation represents an ion originating from a base, or a metal ion, which is pharmaceutically compatible.

2. New halogen derivative of δ-hydroxycarboxylic acids, as claimed in claim 1, which consists of 4-(4′-chlorophenyl)-6-methyl-3-phenyl-3,4,5,6-tetrahydropyrone-2.

3. New halogen derivative of δ-hydroxycarboxylic acids, as claimed in claim 1, which consists of the sodium salt of 3-(4′-chlorophenyl)-5-hydroxy-2-phenylhexanoic acid.

4. Derivatives as claimed in any of claims 1 to 3, which are stereochemically pure.

5. Medicaments having a psychostimulant antidepressive action, which comprise, as active constituents, halogen derivatives of δ-hydroxycarboxylic acids, as claimed in any of claims 1 to 4.

6. Medicaments as claimed in claim 5, which are stereochemically pure.

7. Process for the preparation of the compounds as claimed in claim 1, which comprises preparing a halogenoketonitrile in a first step, and subsequently converting it, by hydrolysis and reduction, into the corresponding compound of the general formula III, which has the open from (a) in a basic medium or the cyclized form (b) in an acid medium.

8. Process as claimed in claim 7, wherein the halogenoketonitrile is first reduced to give the corresponding halogenohydroxynitrile, and the latter is then cyclized and hydrolyzed in the presence of an acid to give the compound of the general formula IIIb.

9. Process as claimed in claim 7, wherein the ketonitrile is first converted into the corresponding halogenoketoacid by hydrolysis in the presence of acid, and the halogenoketoacid is then reduced and cyclized to give a compound of the general formula IIIb.

10. Process as claimed in claim 7, wherein the halogenoketonitrile is first converted into the ketoacid by acid hydrolysis, the ketoacid is then converted into the keto-salt by a pharmaceutically compatible base, and the keto-salt is then reduced to give a compound of the general formula IIIa.

11. Process as claimed in claim 7, wherein the halogenoketonitrile is converted into the ketoester by acid treatment in the presence of an alcohol, the ketoester is then reduced to give the corresponding hydroxyester, and the hydroxyester is finally hydrolyzed by a pharmaceutically compatible base to give a compound of the general formula IIIa.

12. Process as claimed in claim 11, wherein the ketoester is first treated with a pharmaceutically compatible base to give a keto-salt, and the keto-salt is then reduced to give a compound of the general formula IIIa.

13. Process as claimed in claim 7, wherein the halogenoketonitrile is converted into the corresponding keto-salt by alkaline hydrolysis, and the keto-salt is then reduced.

14. Process as claimed in any of claims 7 to 13, wherein the halogenoketonitrile is prepared by reacting an α-ethylenic ketone, which corresponds to the general formula IV below:

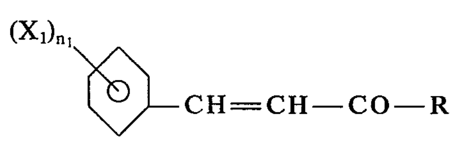

(IV)

with benzyl cyanide of the general formula V below:

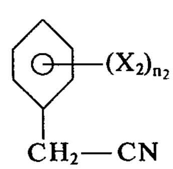

(V)

to give a compound of the general formula VI below:

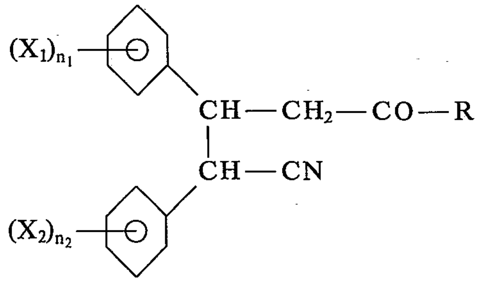

(VI)

in which formulae: $X_1$, $X_2$, $n_1$, $n_2$ and R have the same meaning as above.

15. Process for the preparation of halogen derivatives of δ-hydroxycarboxylic acids, as claimed in any of claims 7 to 14, which comprises carrying out several recrystallizations in suitable solvents, during each synthesis step, in order to separate the trans (threo) derivatives from the cis (erythro) derivatives or to enrich the mixture in one of these trans or cis derivatives.

16. Process as claimed in claim 15, wherein the intermediates consisting of the hydroxynitriles, the ketonitriles, the ketoacids and the ketoesters, and also the pyrones, are recrystallized from polar solvents, the intermediates consisting of the hydroxyesters are recrystallized from polar solvents which do not contain oxygen, and the keto-salts and the hydroxysalts are recrystallized from polar solvents containing at least 10% of water.

17. Process as claimed in any of claims 7 to 14, wherein the cyclization in an acid medium, in order to give the derivatives of the general formula III, takes place at ambient temperature if it is desired to obtain the diastereoisomer of the β-form, or at the reflux temperature if it is desired to obtain the diastereoisomer of the α-form.

18. Process as claimed in any of claims 7 to 17, wherein the reduction of the ketoesters or of the ketosalts to give the corresponding hydroxyesters and hydroxysalts is carried out using a solution of $NaBH_4$ in water.

**Claims for the contracting state AT**

1. Process for the preparation of new halogen derivatives of δ-hydroxycarboxylic acids of the formula IIIa

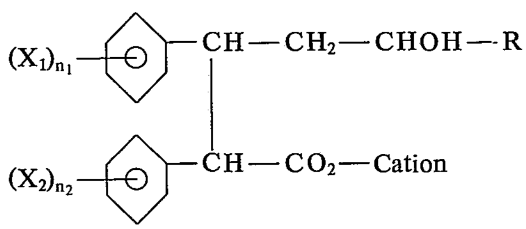

(IIIa)

(open form, in a basic medium)

or of the formula IIIb

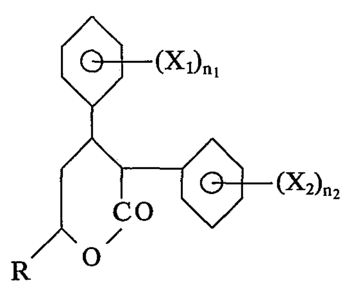

(IIIb)

(cyclized form, in an acid medium)

wherein $X_1$ and $X_2$ are halogen atoms, $n_1$ and $n_2$ are integers between 0 and 5, but, if $n_1 = 0$, $n_2$ is different from 0, and vice versa, R is a hydrogen atom or an alkyl or aryl group, and Cation represents

an ion originating from a base, or a metal ion, which is pharmaceutically compatible, characterized by converting a halogeno-ketonitrile by hydrolysis and reduction into the corresponding compound of formula III, which has the open form (a) in a basic medium or the cyclized form (b) in an acid medium.

2. Process as claimed in claim 1, wherein the halogenoketonitrile is first reduced to give the corresponding halogenohydroxynitrile, and the latter is then cyclized and hydrolyzed in the presence of an acid to give the compound of the general formula IIIb.

3. Process as claimed in claim 1, wherein the ketonitrile is first converted into the corresponding halogenoketoacid by hydrolysis in the presence of acid, and the halogenoketoacid is then reduced and cyclized to give a compound of the general formula IIIb.

4. Process as claimed in claim 1, wherein the halogenoketonitrile is first converted into the ketoacid by acid hydrolysis, the ketoacid is then converted into the keto-salt by a pharmaceutically compatible base, and the keto-salt is then reduced to give a compound of the general formula IIIa.

5. Process as claimed in claim 1, wherein the halogenoketonitrile is converted into the ketoester by acid treatment in the presence of an alcohol, the ketoester is then reduced to give the corresponding hydroxyester, and the hydroxyester is finally hydrolyzed by a pharmaceutically compatible base to give a compound of the general formula IIIa.

6. Process as claimed in claim 5, wherein the ketoester is first treated with a pharmaceutically compatible base to give a keto-salt, and the keto-salt is then reduced to give a compound of the general formula IIIa.

7. Process as claimed in claim 1, wherein the halogenoketonitrile is converted into the corresponding keto-salt by alkaline hydrolysis, and the keto-salt is then reduced.

8. Process as claimed in any of claims 1 to 7, wherein the halogenoketonitrile is prepared by reacting an $\alpha$-ethylenic ketone, which corresponds to the general formula IV below:

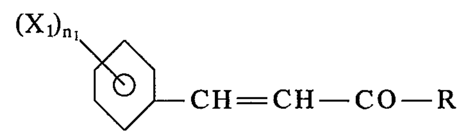

(IV)

with benzyl cyanide of the general formula V below:

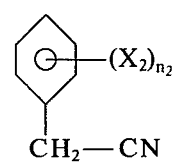

(V)

to give a compound of the general formula VI below:

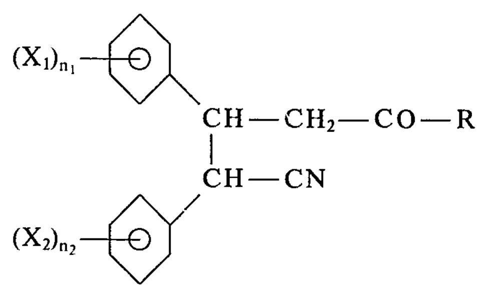

(VI)

in which formulae: $X_1$, $X_2$, $n_1$, $n_2$ and R have the same meaning as above.

9. Process for the preparation of halogen derivatives of $\delta$-hydroxycarboxylic acids, as claimed in any of claims 1 to 8, which comprises carrying out several recrystallizations in suitable solvents, during each synthesis step, in order to separate the trans (threo) derivatives from the cis (erythro) derivatives or to enrich the mixture in one of these trans or cis derivatives.

10. Process as claimed in claim 9, wherein the intermediates consisting of the hydroxynitriles, the ketonitriles, the ketoacids and the ketoesters, and also the pyrones, are recrystallized from polar solvents, the intermediates consisting of the hydroxyesters are recrystallized from polar solvents which do not contain oxygen, and the keto-salts and the hydroxysalts are recrystallized from polar solvents containing at least 10% of water.

11. Process as claimed in any of claims 1 to 8, wherein the cyclization in an acid medium, in order to give the derivatives of the general formula III, takes place at ambient temperature if it is desired to obtain the diastereoisomer of the $\beta$-form, or at the reflux temperature if it is desired to obtain the diastereoisomer of the $\alpha$-form.

12. Process as claimed in any of claims 1 to 11, wherein the reduction of the ketoesters or of the

ketosalts to give the corresponding hydroxyesters and hydroxysalts is carried out using a solution of $NaBH_4$ in water.

**Revendications pour les états contractants BE, CH, DE, FR, GB, IT, NL, SE**

1. Nouveaux dérivés d'acides carboxyliques δ-alcools, halogénés, caractérisés en ce gu'ils répondent à'la formule générale IIIa ci-après:

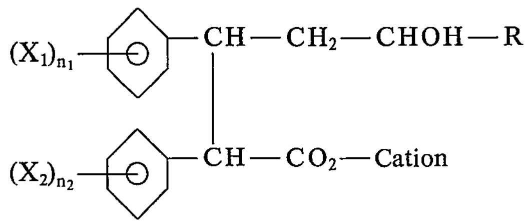

(IIIa)

(forme ouverte, en milieu basique)

ou à la formule générale IIIb ci-après:

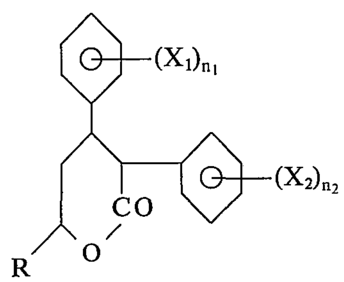

(IIIb)

(forme cyclisée, en milieu acide)

dans lesquelles:
$X_1$ et $X_2$ sont des atomes d'halogène, $n_1$ et $n_2$ sont des nombres entiers compris entre 0 et 5, toutefois, si $n_1 = 0$, $n_2$ est différent de 0 et inversement, R est un atome d'hydrogène, un groupe alkyle ou aryle, Cation représente un ion provenant d'une base ou un ion métallique, pharmaceutiquement compatibles.

2. Nouveau dérivé d'acides carboxyliques δ-alcools, halogéné, selon la Revendication 1, caractérisé en ce qu'il est constitué par la chloro-4' phényl-4 méthyl-6 phényl-3 tétrahydro-3,4,5,6 pyrone-2.

3. Nouveau dérivé d'acides carboxyliques δ-alcools, halogéné, selon la Revendication 1, caractérisé en ce qu'il est constitué par le sel de sodium de l'acide chloro-4' phényl-3 hydroxy-5 phényl-2 hexanoïque.

4. Dérivés selon l'une quelconque des Revendications 1 à 3, caractérisés en ce qu'ils sont stéréochimiquement purs.

5. Médicaments à action antidépressive psychostimulante, caractérisés en ce qu'ils comprennent, en tant que constituants actifs, des dérivés d'acides carboxyliques δ-alcools, halogénés, selon l'une quelconque des Revendications 1 à 4.

6. Médicaments selon la Revendication 5, caractérisés en ce qu'ils sont stéréochimiquement purs.

7. Procédé pour la préparation des composés selon la Revendication 1, caractérisé en ce que l'on prépare au cours d'une première étape, un cétonitrile halogéné, que l'on transforme ensuite par hydrolyse et réduction en le composé de formule générale III correspondant, de forme ouverte (a) en milieu basique ou de forme cyclisée (b) en milieu acide.

8. Procédé selon la Revendication 7, caractérisé en ce que le cétonitrile halogéné est d'abord réduit en hydroxy-nitrile halogéné correspondant, puis l'on cyclise et hydrolyse en présence d'un acide, pour obtenir le composé de formule générale IIIb.

9. Procédé selon la Revendication 7, caractérisé en ce que le cétonitrile est tout d'abord transformé en cétoacide halogéné correspondant par hydrolyse en présence d'acide, puis l'on procède à la reduction et à la cyclisation pour obtenir un composé de formule générale IIIb.

10. Procédé selon la Revendication 7, caractérisé en ce que le cétonitrile halogéné est d'abord transformé en cétoacide par hydrolyse acide, puis transformé en céto-sel par une base pharmaceutiquement compatible, puis l'on procède à la réduction pour obtenir un composé de

formule générale IIIa.

11. Procédé selon la Revendication 7, caractérisé en ce que le cétonitrile halogéné est transformé en cétoester par traitement acide en présence d'un alcool, puis il est réduit en l'hydroxyester correspondant, enfin l'on hydrolyse par une base pharmaceutiquement compatible, pour obtenir un composé de formule générale IIIa.

12. Procédé selon la Revendication 11, caractérisé en ce que le cétoester est d'abord traité par une base pharmaceutiquement compatible pour obtenir un céto-sel, puis l'on procède à la réduction pour obtenir un composé de formule générale IIIa.

13. Procédé selon la Revendication 7, caractérisé en ce que le cétonitrile halogéné est transformé en le céto-sel correspondant par hydrolyse alcaline, puis l'on procède à la réduction.

14. Procédé selon l'une quelconque des Revendications 7 à 13, caractérisé en ce que le cétonitrile halogéné est préparé par action d'une cétone $\alpha$-éthylénique qui répond à la formule générale IV ci-après:

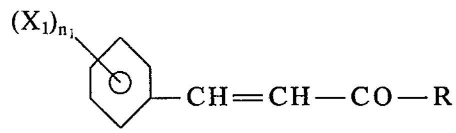

(IV)

sur le cyanure benzylique de formule générale V ci-après:

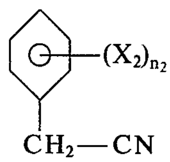

(V)

pour obtenir un composé de formule générale VI ci-après:

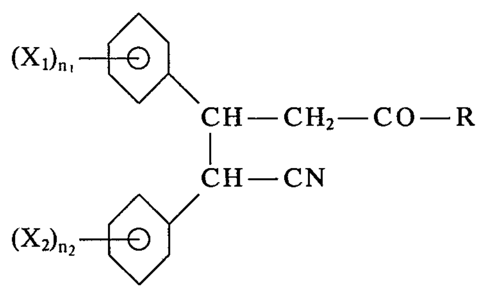

(VI)

dans lesquelles:
$X_1$, $X_2$, $n_1$, $n_2$ et R ont la même signification que ci-dessus.

15. Procédé de préparation de dérivés d'acides carboxyliques $\delta$-alcools, halogénés, selon l'une quelconque des Revendications 7 à 14, caractérisé en ce qu'au cours de chaque étape de synthèse, on procède à plusieurs recristallisations dans des solvants appropriés, en vue de séparer les dérivés trans (thréo) des dérivés cis (érythro) ou d'enrichir le mélange en l'un de ces dérivés trans ou cis.

16. Procédé selon la Revendication 15, caractérisé en ce que les produits intermédiaires constitués par les hydroxynitriles, les cétonitriles, les cétoacides, les cétoesters, ainsi que les pyrones sont recristallisés dans des solvants polaires, les produits intermédiaires constitués par les hydroxyesters sont recristallisés dans des solvants polaires non oxygénés, tandis que les cétosels et les hydroxysels sont recristallisés dans des solvants polaires contenant au moins 10% d'eau.

17. Procédé selon l'une quelconque des Revendications 7 à 14, caractérisé en ce que la cyclisation en milieu acide pour obtenir les dérivés de formule générale III, a lieu à la température ambiante si c'est le diastéréoisomère de forme $\beta$ qui est recherché ou à la température du reflux si c'est l'obtention du diastéréoisomère de forme $\alpha$ qui est recherchée.

18. Procédé selon l'une quelconque des Revendications 7 à 17, caractérisé en ce que la réduction des cétoesters ou des cétosels en hydroxyesters et hydroxysels correspondants est effectuée à l'aide du $NaBH_4$ en solution dans de l'eau.

**Revendications pour l'Etat AT**

1. Procédé de préparation de nouveaux dérivés d'acides carboxyliques $\delta$-alcools, halogénés répondant à la formule générale IIIa ci-après:

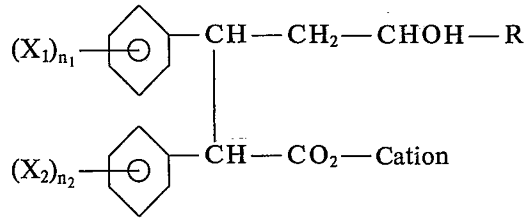

(IIIa)

(forme ouverte, en milieu basique)

ou à la formule générale IIIb ci-après:

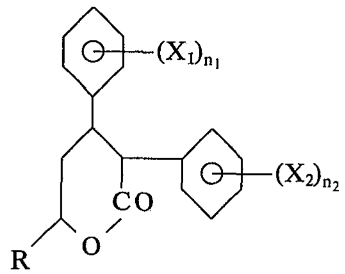

(IIIb)

(forme cyclisée, en milieu acide)

dans lesquelles:
$X_1$ et $X_2$ sont des atomes d'halogène, $n_1$ et $n_2$ sont des nombres entiers compris entre 0 et 5, toutefois, si $n_1 = 0$, $n_2$ est différent de 0 et inversement, R est un atome d'hydrogène, un groupe alkyle ou aryle, Cation représente un ion provenant d'une base ou un ion métallique, pharmaceutiquement compatible, caractérisé en ce que l'on transforme un cétonitrile halogéné par hydrolyse et réduction en le composé de formule générale III correspondant, de forme ouverte (a) en milieu basique ou de forme cyclisée (b) en milieu acide.

2. Procédé selon la Revendication 1, caractérisé en ce que le cétonitrile halogéné est d'abord réduit en hydroxynitrile halogéné correspondant, puis l'on cyclise et hydrolyse en présence d'un acide, pour obtenir le composé de formule générale IIIb.

3. Procédé selon la Revendication 1, caractérisé en ce que le cétonitrile est tout d'abord transformé en céto-acide halogéné correspondant par hydrolyse en présence d'acide, puis l'on procède à la réduction et à la cyclisation pour obtenir un composé de formule générale IIIb.

4. Procédé selon la Revendication 1, caractérisé en ce que le cétonitrile halogéné est d'abord transformé en céto-acide par hydrolyse acide, puis transformé en céto-sel par une base pharmaceutiquement compatible, puis l'on procède à la réduction pour obtenir un composé de formule générale IIIa.

5. Procédé selon la Revendication 1, caractérisé en ce que le cétonitrile halogéné est transformé en cétoester par traitement acide en présence d'un alcool, puis il est réduit en l'hydroxyester correspondant, enfin l'on hydrolyse par une base pharmaceutiquement compatible, pour obtenir un composé de formule générale IIIa.

6. Procédé selon la Revendication 5, caractérisé en ce que le cétoester est d'abord traité par une base pharmaceutiquement compatible pour obtenir un céto-sel, puis l'on procède à la réduction pour obtenir un composé de formule générale IIIa.

7. Procédé selon la Revendication 1, caractérisé en ce que le cétonitrile halogéné est transformé en le céto-sel correspondant par hydrolyse alcaline, puis l'on procède à la réduction.

8. Procédé selon l'une quelconque des Revendications 1 à 7, caractérisé en ce que le cétonitrile halogéné est préparé par action d'une cétone $\alpha$-éthylénique qui répond à la formule générale IV ci-après:

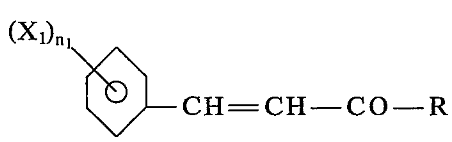

(IV)

sur le cyanure benzylique de formule générale V ci-après:

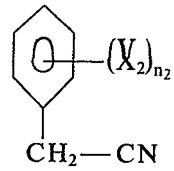

(V)

pour obtenir un composé de formule générale VI ci-après:

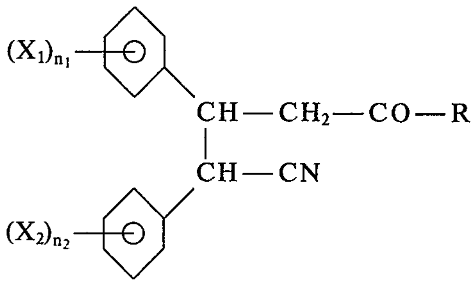

(VI)

dans lesquelles:

$X_1$, $X_2$, $n_1$, $n_2$ et R ont la même signification que ci-dessus.

9. Procédé de préparation de dérives d'acides carboxyliques $\delta$-alcools, halogénés, selon l'une quelconque des Revendications 1 à 8, caractérisé en ce qu'au cours de chaque étape de synthèse, on procède à plusieurs recristallisations dans des solvants appropriés, en vue de séparer les dérivés trans (thréo) des dérivés cis (érythro) ou d'enrichir le mélange en l'un de ces dérivés trans ou cis.

10. Procédé selon la Revendication 9, caractérisé en ce que les produits intermédiaires constitués par les hydroxynitriles, les cétonitriles, les cétoacides on les cétoesters, ainsi que les pyrones sont recristallisés dans des solvants polaires, les produits intermédiaires constitués par les hydroxyesters sont recristallisés dans des solvants polaires non oxygénés, tandis que les cétosels et les hydroxysels sont recristallisés dans des solvants polaires contenant au moins 10% d'eau.

11. Procédé selon l'une quelconque des Revendications 1 à 8, caractérisé en ce que la cyclisation en milieu acide pour obtenir les dérivés de formule générale III, a lieu à la température ambiante si c'est le diastéréoisomère de forme $\beta$ qui est recherché ou à la température du reflux si c'est l'obtention du diastéréoisomère de forme $\alpha$ qui est recherchée.

12. Procédé selon l'une quelconque des Revendications 1 à 11, caractérisé en ce que la réduction des cétoesters ou des cétosels en hydroxyesters et hydroxysels correspondants est effectuée à l'aide du $NaBH_4$ en solution dans de l'eau.

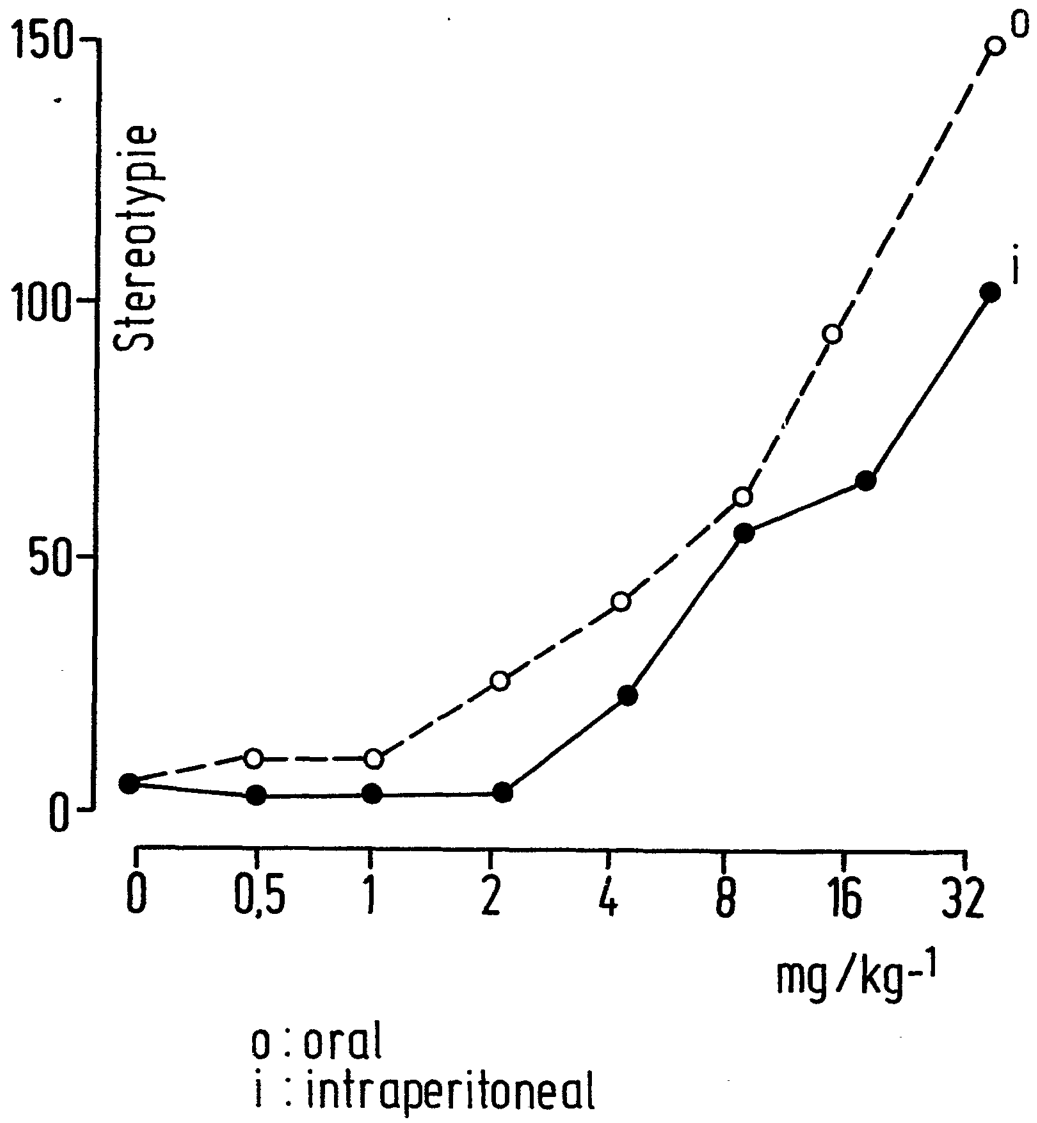
150
100
50
0
Stereotypie
o
i
0
0,5
1
2
4
8
16
32
mg/kg-1
o : oral
i : intraperitoneal